# EUROPEAN PATENT APPLICATION

(11) **EP 4 613 875 A1**
(43) Date of publication of application: **10.09.2025**
(21) Application number: 23927951.6
(22) Date of filing: 20.03.2023
(51) Int. Cl.: C12Q 1/6855, C12P 19/34, C07H 21/00

(54) **METHOD FOR EFFICIENTLY AND QUICKLY CONSTRUCTING NUCLEIC ACID LIBRARY**

(71) Applicant: MGI Tech Co., Ltd., Shenzhen, Guangdong 518083 (CN)
(72) Inventor: XIA, Jun, Shenzhen, Guangdong 518083 (CN); KONG, Gezhi, Shenzhen, Guangdong 518083 (CN); YANG, Lin, Shenzhen, Guangdong 518083 (CN); SHI, Keke, Shenzhen, Guangdong 518083 (CN); ZHANG, Yanyan, Shenzhen, Guangdong 518083 (CN); LIU, Ping, Shenzhen, Guangdong 518083 (CN); WANG, Yicong, Shenzhen, Guangdong 518083 (CN); ZHAO, Xia, Shenzhen, Guangdong 518083 (CN); CHEN, Fang, Shenzhen, Guangdong 518083 (CN)
(74) Representative: Boco IP Oy Ab
(86) International application number: PCT/CN2023/082542
(87) International publication number: WO 2024/192634

(57) **Abstract**

The present invention relates to the technical field of library construction, and particularly relates to a method for efficiently and quickly constructing a nucleic acid library. The present invention provides an adaptor composition. The adaptor composition can simultaneously attach a 5' end adaptor and a 3' end adaptor to a single-stranded template nucleic acid in a same reaction system by means of the 5' end adaptor, a first ligase, the 3' end adaptor, and a second ligase, without the need to add the 5' end adaptor and the 3' end adaptor in separate steps, thereby greatly reducing the reaction steps and the library construction time, and improving the adaptor attachment efficiency; moreover, the adaptor attachment directionality can be guaranteed, the template nucleic acid loss is reduced, and the utilization rate of original template nucleic acids is increased; no additional fixed sequence is added onto the template, the sequencing quality is not affected, there is no need to perform additional truncation by a certain length of a sequence requiring sequencing, and adaptors will not be attached to each other.

## Description

### TECHNICAL FIELD

The present disclosure belongs to the technical field of library construction, and particularly relates to a method for efficiently and quickly constructing a nucleic acid library.

### BACKGROUND

High-throughput sequencing has become one of the most important research methods in life sciences and has been widely used in various fields such as clinical medicine, forensics, and ancient DNA research. High-throughput sequencing requires the construction of sequencing library, with the most common being DNA sequencing library, which commonly targets at intact genomic DNA with a sufficient starting amount. For such samples, conventional double-stranded DNA library construction methods may be used, of which the general steps involve fragmenting the DNA into the desired size, repairing the ends of the fragmented DNA, and then ligating the adapters to two ends of the DNA template fragment through a simple ligase reaction to obtain the library for sequencing. However, besides the common intact DNA samples, there are some special samples, such as formalin-fixed paraffin-embedded (FFPE) biological tissue samples, forensic samples, and paleontological fossils. The DNA extracted from these samples may undergo severe degradation, and there may exist both double-stranded and single-stranded DNA molecules, or there may be single-strand breakage in double-stranded DNA molecules. In addition, the amount of DNA extracted from these samples is often much lower than that from conventional samples. Moreover, in some special applications, such as DNA methylation sequencing, the DNA needs to undergo bisulfite treatment, and after the treatment, the DNA would break into short fragments, resulting in the loss of the vast majority of DNA molecules and the formation of single-stranded molecules. The extracellular free DNAs in plasma or other bodily fluid samples have fragments that are already short in length and present in very low quantities. For these special samples, common library construction methods with adapter ligation may not be applicable, because the conventional methods can only be applied for intact double-stranded DNA molecules, which would result in a loss of a large number of original template molecules, as all template molecules other than intact double-stranded DNA molecules would be unable to or only rarely be ligated with adapters.

Single-stranded nucleic acid library construction technologies may be able to utilize these single-stranded DNA template molecules, by adding adapters to these single-stranded DNA molecules, and the double-stranded DNA can also be treated in the same way after denaturation into single-stranded DNA for adding adapters. It may be conducive to avoid the loss of these irregular double-stranded DNA template molecules to the greatest extent, maximize the utilization rate of templates, increase the efficiency of library construction, and improve the effective data volume and data quality of sequencing. This can also facilitate samples to be better utilized for corresponding application detection, such as extracellular free DNA (cfDNA) methylation sequencing of liquid biopsy, genomic research of ancient biological DNA, forensic identification, tumor detection of FFPE samples, etc. In addition to DNA samples, single-stranded nucleic acid library construction technologies are also very suitable for RNA samples, as RNA itself is single-stranded. In general library construction methods, RNA is first reverse transcribed into double-stranded cDNA, and then adapters are added to the cDNA. Single-stranded library construction technologies can also be used to directly add adapters to RNA samples, which are then reverse transcribed into cDNA, to complete the construction of sequencing library.

Existing single-stranded nucleic acid library construction technologies include conventional single-stranded nucleic acid library construction method, Swift's Adaptase single-stranded nucleic acid library construction technology, random primer-based adapter ligation method, terminal transferase-based adapter ligation method, and Splinted adapter tagging (SPALT) technology.

Conventional single-stranded nucleic acid library construction method: Based on T4 RNA ligase, the single-stranded DNA template molecule is directly ligated with a single-stranded adapter. Generally, T4 RNA ligase 2 (truncated type) is used to catalyze the ligation of a 5' end pre-adenylated and 3' end closed DNA adapter to the single-stranded template at the 3' end. After the 3' end adapter is ligated, the adapter at the 3' end needs to be digested to prevent the self-ligation with the subsequent adapter. Then a 5' end adapter and a T4 RNA ligase are added for the ligation of the 5' end adapter to the nucleic acid fragment that has already been ligated with the 3' end adapter. After both the end adapters are added, PCR amplification is performed. If the previously ligated adapter is truncated, PCR also has the function of completing the adapter.

Swift's Adaptase single-stranded nucleic acid library construction technology is to first ligate the 3' end of the single-stranded DNA template with a universal adapter bearing a random primer through a single-stranded nucleic acid ligase, and then perform amplification using a complementary primer that binds to the sequence of the 3' end adapter primer, thereby converting the single-stranded template into a double-stranded one. Finally, through a conventional adapter ligation reaction, a 5' end adapter is added to the 5' end of the newly synthesized strand, so as to complete the construction of the library.

Random primer-based single-stranded nucleic acid library construction technology involves binging a 6-8 nt random primer with adapter sequence at the 3' end to a single-stranded DNA template, and extending the complementary strand, thereby synthesizing a double-stranded DNA. Then, a 5' end adapter is added to the 5' end of the newly synthesized strand, so as to complete the library construction.

Similar to the random primer-based technology, terminal transferase-based single-stranded nucleic acid library construction technology involves first adding continuous identical bases to the 3' end of the single-stranded DNA template using terminal transferase, then using the continuous sequence as the primer binding sequence to hybridize with a primer, and then preforming extension to convert the single-stranded DNA template into a double-stranded DNA, and then adding a 5' end adapter to the 5' end of the newly synthesized strand to complete the library construction.

SPALT (Splinted adapter tagging) technology involves adding a double-stranded adapter, which contains a segment of random bases for complementary binding with a single-stranded DNA, to the 3' end of single-stranded DNA; then adding a double-stranded adapter with random bases to the 5' end of the single-stranded template DNA; and after adding adapters to both ends of the single-stranded template, performing PCR amplification, so as to complete the construction of the single-stranded library.

The conventional single-stranded nucleic acid library construction method involves connecting the adapters to the two ends separately, and requires multiple purifications, resulting in low template utilization and long library construction time.

Swift's Adaptase, random primer-based and terminal transferase-based single-stranded nucleic acid library construction technologies, as well as SPALT technology, all involve adding the 3' and 5' adapters separately, and only after adding the adapters to both ends can PCR amplification be performed to amplify the template. All of these increase the experimental steps, leading to the loss of the original template and the decrease of the utilization rate. **In** addition, the Swift's Adaptase and the terminal transferase-based single-stranded nucleic acid library construction technologies require adding a fixing sequence to the template, which may impair the sequencing quality, and after obtaining the sequencing data, these fixing sequences need to be removed, which may in turn result in data loss.

### SUMMARY

In accordance to a first aspect of the present disclosure, provided is an adapter composition.

In accordance to a second aspect of the present disclosure, provided is a kit for nucleic acid library construction.

In accordance to a third aspect of the present disclosure, provided is a sequencing reagent kit.

In accordance to a fourth aspect of the present disclosure, provided is a sequencing system.

In accordance to a fifth aspect of the present disclosure, provided is a method for adapter ligation.

In accordance to a sixth aspect of the present disclosure, provided is a method for nucleic acid library construction.

In accordance to a seventh aspect of the present disclosure, provided is a nucleic acid library.

In accordance to an eighth aspect of the present disclosure, provided is a sequencing method.

In accordance to a ninth aspect of the present disclosure, provided is use of the adapter composition of the first aspect, the kit of the second aspect, the sequencing reagent kit of the third aspect, or the sequencing system of the fourth aspect.

In order to achieve the above objectives, the present disclosure uses the following technical solutions.

In accordance to a first aspect of the present disclosure, provided is an adapter composition, which includes:
a 5' end adapter, including a first oligonucleotide, wherein the first oligonucleotide of the 5' end adapter has a first modifying group at the 3' end, the first oligonucleotide of the 5' end adapter has a first blocking group at the 5' end, and the first modifying group includes a phosphate group (3'-phosphate group); and
a first ligase, wherein the first ligase is capable of ligating the first modifying group at the 3' end of the first oligonucleotide of the 5' end adapter to a 5'-hydroxyl end (5'-OH) of a template nucleic acid (i.e., ligating the 3'-phosphate group of the first modifying group to the 5'-OH); and
a 3' end adapter, including a second oligonucleotide, wherein the second oligonucleotide of the 3' end adapter has a second blocking group at the 3' end, the second oligonucleotide of the 3' end adapter has a second modifying group at the 5' end, and the second modifying group includes a phosphate group (5'-phosphate group); and
a second ligase, wherein the second ligase is capable of ligating the second modifying group at the 5' end of the second oligonucleotide of the 3' end adapter to a 3'-hydroxyl end (3'-OH) of the template nucleic acid (i.e., ligating the 5'-phosphate group of the second modifying group to the 3'-OH).

Preferably, the first modifying group is at least one modification selected from the group consisting of phosphorylation modification, 2'3' cyclic phosphate modification, and pre-guanosine modification, and the like.

Preferably, the second modifying group is at least one modification selected from the group consisting of phosphorylation modification and adenylation modification.

Preferably, the 5' end adapter may further include a third oligonucleotide, wherein the third oligonucleotide is in complementary with the first oligonucleotide to form a double strand. And a length of the third oligonucleotide is greater than, equal to or less than a length of the first oligonucleotide.

Preferably, the difference between the base numbers of the third oligonucleotide and the first oligonucleotide is greater than or equal to 2.

Preferably, the third oligonucleotide has a third blocking group at the 5' end, and the third oligonucleotide has a fourth blocking group at the 3' end.

Preferably, the 3' end adapter may further include a fourth oligonucleotide, wherein the fourth oligonucleotide is in complementary with the second oligonucleotide to form a double strand.

Preferably, a length of the fourth oligonucleotide is greater than, equal to or less than a length of the second oligonucleotide.

Preferably, the fourth oligonucleotide has a fifth blocking group at the 5' end, and the fourth oligonucleotide has a sixth blocking group at the 3' end.

Preferably, the first ligase is at least one selected from the group consisting of a RtcB ligase, an active fragment of RtcB ligase, a derivative of RtcB ligase, and an analog of RtcB ligase.

Preferably, the adapter composition may further include: a first cofactor including triphosphate and manganese ion.

Preferably, the adapter composition may further include: a second cofactor including at least one of ATP and NAD, and a metal ion.

Preferably, when the 5' end of the second oligonucleotide of the 3' end adapter is phosphorylated (i.e., has a phosphorylation modification), the second ligase includes at least one of T4 RNA ligase 1, T4 DNA ligase, TS2126 RNA ligase, T3 DNA ligase, E. coli DNA ligase, single-stranded DNA/RNA circligase (ssDNA/RNA CircLigase), T4 RNA ligase 2, and Taq DNA ligase.

Preferably, when the 5' end of the second oligonucleotide of the 3' end adapter is adenylated (i.e., has an adenylation modification), the second ligase includes at least one of T4 RNA ligase 1, T4 RNA ligase 2, T4 RNA ligase 2 (truncated K227Q), T4 RNA ligase 2 (truncated KQ), thermostable 5' App DNA/RNA ligase, TS2126 RNA ligase, T3 DNA ligase, E. coli DNA ligase, single-stranded DNA/RNA circligase (ssDNA/RNA CircLigase), and Taq DNA ligase.

Preferably, the first blocking group is a modification selected from the group consisting of amino modification, dideoxy modification, spacer modification, and phosphate modification.

Preferably, the second blocking group is a modification selected from the group consisting of amino modification, dideoxy modification, spacer modification, and phosphate modification.

Preferably, the third blocking group is a modification selected from the group consisting of amino modification, dideoxy modification, spacer modification, and phosphate modification.

Preferably, the fourth blocking group is a modification selected from the group consisting of amino modification, dideoxy modification, spacer modification, and phosphate modification.

Preferably, the fifth blocking group is a modification selected from the group consisting of amino modification, dideoxy modification, spacer modification, and phosphate modification.

Preferably, the sixth blocking group is a modification selected from the group consisting of amino modification, dideoxy modification, spacer modification, and phosphate modification.

Preferably, the adapter composition may further include a phosphatase.

Preferably, the adapter composition is used for ligating the 5' end adapter to the 5' end of the single-stranded template nucleic acid and the 3' end adapter to the 3' end of the single-stranded template nucleic acid.

Preferably, the adapter composition is used for ligating the 5' end adapter and the 3' end adapter respectively to the 5' end and 3' end of the single-stranded template nucleic acid in a same system; wherein the same system specifically refers to carrying out the reaction in a same reaction vessel; and further specifically, to carrying out the reaction in the same reaction vessel at the same time.

Preferably, the single-stranded template nucleic acid is obtained by subjecting the nucleic acid to at least one treatment of fragment, denaturation, and DNA methylation.

Preferably, the nucleic acid includes at least one of DNA and RNA.

Preferably, the DNA includes at least one of dsDNA and ssDNA.

Preferably, the nucleic acid is derived from the following biological samples: cells, fresh tissues, fresh organs, decayed tissues, formalin-fixed tissues, paraffin-embedded tissues, forensic samples, paleontological fossils, or biological materials containing cfDNA or RNA.

Preferably, the adapter composition may further include a substance for nucleic acid fragment.

Preferably, the adapter composition may further include a substance for nucleic acid denaturation.

Preferably, when the adapter composition is used for constructing a DNA methylation library, the adapter composition may further include a DNA methylation modification conversion reagent, which is capable of converting non-methylated C bases in DNA into U bases.

Preferably, the first oligonucleotide of the 5' end adapter is different from the second oligonucleotide of the 3' end adapter.

Preferably, the first oligonucleotide of the 5' end adapter and/or the second oligonucleotide of the 3' end adapter is immobilized on a solid phase support.

Preferably, a non-ligating end of the first oligonucleotide of the 5' end adapter and/or the second oligonucleotide of the 3' end adapter is immobilized on the solid phase support.

Preferably, the solid phase support is selected from the group consisting of a bead, a chip, etc.

Preferably, the chip may be one that is usable in a sequencing platform, for example, a sequencing chip.

Preferably, the bead is selected from the group consisting of agarose gel beads, agarose beads, magnetic beads, protein A conjugated beads, protein G conjugated beads, protein L conjugated beads, oligo (dT) conjugated beads, silica beads, hydrogel beads, silica-like beads, anti-biotin microbeads, anti-fluorescent dye microbeads, and any combination thereof.

In accordance to a second aspect of the present disclosure, provided is a kit for nucleic acid library construction, which includes: the adapter composition of the first aspect of the present disclosure.

Preferably, the kit for nucleic acid library construction may further include a polynucleotide kinase.

Preferably, the kit for nucleic acid library construction may further include a PCR primer, which is used for PCR amplification of a single-stranded template nucleic acid ligated with adapters.

Preferably, the kit for nucleic acid library construction may further include a PCR reaction mixture.

Preferably, the kit for nucleic acid library construction may further include a reverse transcription primer, which is used for reverse transcription of RNA into cDNA when the nucleic acid contains RNA.

Preferably, the kit for nucleic acid library construction may further include a reverse transcription reaction solution.

In accordance to a third aspect of the present disclosure, provided is a sequencing reagent kit, which includes: any one of a1) and a2):
a1) the adapter composition of the first aspect of the present disclosure; and
a2) the kit for nucleic acid library construction of the second aspect of the present disclosure.

Preferably, the sequencing reagent kit may further include a sequencing kit.

In accordance to a fourth aspect of the present disclosure, provided is a sequencing system, which includes: any one of a1) to a3), and a sequencer:
a1) the adapter composition of the first aspect of the present disclosure;
a2) the kit for nucleic acid library construction of the second aspect of the present disclosure; and
a3) the sequencing reagent kit of the third aspect of the present disclosure.

In accordance to a fifth aspect of the present disclosure, provided is a method for adapter ligation, which includes the step of using the adapter composition of the first aspect of the present disclosure.

Preferably, the method for adapter ligation includes the following steps: obtaining a single-stranded template nucleic acid, wherein the 5' end and the 3' end of the single-stranded template nucleic acid are both hydroxyl groups; and performing an adapter ligation reaction of a 5' end adapter and a 3' end adapter to the single-stranded template nucleic acid using the first ligase, the 5' end adapter, the second ligase and the 3' end adapter in the adapter composition of the first aspect of the present disclosure.

Preferably, the ligation reaction is performed in the same system.

Preferably, the same system specifically refers to carrying out the reaction in the same reaction vessel; and further specifically, to carrying out the reaction in the same reaction vessel at the same time.

Preferably, the adapter ligation is directional, and the first oligonucleotide of the 5' end adapter has a different nucleotide sequence from the second oligonucleotide of the 3' end adapter.

Preferably, the single-stranded template nucleic acid with hydroxyl groups at both the 5' end and the 3' end is obtained by dephosphorylation treatment.

Preferably, the single-stranded template nucleic acid is obtained by subjecting the nucleic acid to at least one treatment of fragment, denaturation, and DNA methylation.

Preferably, the nucleic acid includes at least one of DNA and RNA.

Preferably, the DNA includes at least one of dsDNA and ssDNA.

Preferably, the nucleic acid include: at least one of dsDNA, ssDNA, and RNA.

Preferably, the nucleic acid is derived from the following biological samples: cells, fresh tissues, fresh organs, decayed tissues, formalin-fixed tissues, paraffin-embedded tissues, forensic samples, paleontological fossils, or biological materials containing cfDNA or cfRNA.

Preferably, the biological materials include but are not limited to: peripheral blood, plasma, serum, urine, feces, saliva, cerebrospinal fluid, lymph fluid, bronchoalveolar lavage fluid, amniotic fluid, blastocyst cavity fluid, cell culture fluid, embryo culture fluid, microbial culture medium, soil leachate, or bone meal leachate.

Preferably, when the nucleic acid contains dsDNA, a process of preparing the single-stranded template nucleic acid may include denaturation treatment.

Preferably, the denaturation treatment may be performed before or after the phosphorylation treatment.

Preferably, when the nucleic acid is derived from the following biological samples: cells, fresh tissues, fresh organs, paraffin-embedded tissues, or forensic samples, the process of preparing the single-stranded template nucleic acid may include fragment treatment.

Preferably, the fragment treatment is performed before the phosphorylation treatment and the denaturation treatment.

Preferably, when the nucleic acid is used for constructing a DNA methylation library, the process of preparing the single-stranded template nucleic acid may include DNA methylation treatment.

Preferably, the DNA methylation treatment is performed before the phosphorylation treatment and the denaturation treatment.

Preferably, the DNA methylation treatment is performed after the fragment treatment.

In accordance to a sixth aspect of the present disclosure, provided is a method for nucleic acid library construction, which includes the steps of the method for adapter ligation of the fifth aspect of the present disclosure.

Preferably, for a method for nucleic acid library construction with a PCR-free process, the method may further include the following steps: dephosphorylating the 3' end of the single-stranded template nucleic acid ligated with the adapters, and performing a cyclization reaction to obtain a single-strand cyclization library.

Preferably, for a method for nucleic acid library construction with a PCR process, the method may further include the step of performing an amplification reaction to obtain a nucleic acid library.

Preferably, the amplification reaction is a rolling circle amplification or a linear amplification.

Preferably, the amplification reaction involves using the PCR primer in the kit for nucleic acid library construction of the second aspect of the present disclosure.

Preferably, when the nucleic acid contains RNA, the method for nucleic acid library construction may further include the step of performing a reverse transcription reaction; wherein the reverse transcription reaction may be performed before the adapter ligation reaction or after the adapter ligation reaction.

Preferably, the reverse transcription reaction involves using the reverse transcription primer in the kit for nucleic acid library construction of the second aspect of the present disclosure.

Preferably, the method for nucleic acid library construction may further include the step of purifying the amplified product.

Preferably, the purification is performed using magnetic beads.

Preferably, the method for nucleic acid library construction may further include the step of performing a cyclization reaction after the adapter ligation reaction.

Preferably, the nucleic acid library may include DNA methylation library.

In accordance to a seventh aspect of the present disclosure, provided is a nucleic acid library obtained through the method for nucleic acid library construction of the sixth aspect of the present disclosure.

In accordance to an eighth aspect of the present disclosure, provided is a sequencing method, which includes the steps of the method for nucleic acid library construction of the sixth aspect of the present disclosure.

Preferably, the sequencing method includes the following steps: obtaining a nucleic acid library; and sequencing.

The method of obtaining a nucleic acid library is the method for nucleic acid library construction of the sixth aspect of the present disclosure.

Preferably, the sequencing method may further include a step of library quality inspection before the sequencing.

In accordance to a ninth aspect of the present disclosure, provided is use of the adapter composition of the first aspect of the present disclosure, the kit of the second aspect, the sequencing reagent kit of the third aspect, and/or the sequencing system of the fourth aspect.

The adapter composition of the first aspect of the present disclosure and/or the kit of the second aspect of the present disclosure for use in any one of c1) to c6):
c1) Preparation of a nucleic acid library;
c2) Preparation of a product for nucleic acid library construction;
c3) Sequencing;
c4) Preparation of a product for sequencing;
c5) DNA methylation detection; and
c6) Preparation of a product for DNA methylation detection.

Preferably, the nucleic acid library may include DNA methylation library.

The sequencing reagent kit of the third aspect of the present disclosure and/or the sequencing system of the fourth aspect of the present disclosure for use in any one of c3) to c6):
c3) Sequencing;
c4) Preparation of a product for sequencing;
c5) DNA methylation detection; and
c6) Preparation of a product for DNA methylation detection.

Preferably, the product includes at least one of a reagent, a kit, and a system.

The present disclosure achieves the following beneficial effects.

The present disclosure provides an adapter composition, which can ligate the 5' end adapter to the 5' end of a single-stranded template nucleic acid through the first oligonucleotide of the 5' end adapter and the first ligase, and ligate the 3' end adapter to the 3' end of the single-stranded template nucleic acid through the second oligonucleotide of the 3' end adapter and the second ligase, such that both the 5' end adapter and the 3' end adapter can be ligated respectively to the 5' end and 3' end of the single-stranded template nucleic acid in the same reaction system simultaneously without the need to add the 5' end adapter and 3' end adapter in separate steps, thereby greatly reducing the reaction steps and library construction time, increasing the efficiency of adapter ligation, ensuring the directionality of adapter ligation, reducing the loss of template nucleic acid, and improving the utilization rate of the original template nucleic acid. In addition, no additional fixing sequence needs to be added to the template, the sequencing quality will not be impaired, and there is no need to perform additional truncation by a certain length of the sequenced reads. Furthermore, the adapters are prevented from ligating with each other at all. The technical solution of the present disclosure is suitable for the construction of nucleic acid (including RNA) library (including DNA methylation library) of samples with nucleic acid degradation, such as formalin-fixed tissues, paraffin-embedded tissues, forensic samples, decayed tissues, ancient biological fossils, as well as biological samples containing cfDNA or cfRNA.

Further, the adapter composition may include a phosphatase to prevent the self-ligation between samples.

### BRIEF DESCRIPTION OF DRAWINGS

Figure 1 is a schematic diagram of a method for nucleic acid library construction (with PCR) of the present disclosure.
Figure 2 is a flow diagram of a method for nucleic acid library construction (with PCR) of the present disclosure.
Figure 3 is a diagram indicating the proportion of each base type of the sequenced reads detected in each sequencing cycle in Example 1.
Figure 4 is a flow diagram of a method for nucleic acid library construction (without PCR) of the present disclosure.
Figure 5 is a schematic diagram indicating the ligation principle of RtcB Ligase.

### DETAILED DESCRIPTION

In the description of the present disclosure, the terms "first", "second", "third", "fourth", "fifth", and "sixth" are used only for illustration purposes and should not be understood as indicating or implying relative importance or implicitly indicating a specific quantity of the referred technical features. Therefore, the technical features defined as "first" or "second" may explicitly or implicitly include at least one of the referred features. In the description of the present disclosure, the terms such as "a plurality of", "multiple" and the like refer to at least two, for example, two, three and the like, unless otherwise explicitly and specifically defined.

According to a first aspect of the present disclosure, provided is an adapter composition, which includes:
5' end adapter, including a first oligonucleotide, wherein the first oligonucleotide of the 5' end adapter has a first modifying group at the 3' end, the first oligonucleotide of the 5' end adapter has a first blocking group at the 5' end, and the first modifying group includes a phosphate group (3'-phosphate group); and
a first ligase, wherein the first ligase is capable of ligating the first modifying group at the 3' end of the first oligonucleotide of the 5' end adapter to a 5'-hydroxyl end (5'-OH) of an oligonucleotide (i.e., ligating the 3'-phosphate group of the first modifying group to the 5'OH), that is, the first ligase possesses the ligase activity of ligating the 3'-phosphate group of the first modifying group to the 5' OH; and
3' end adapter, including a second oligonucleotide, wherein the second oligonucleotide of the 3' end adapter has a second blocking group at the 3' end, the second oligonucleotide of the 3' end adapter has a second modifying group at the 5' end, and the second modifying group includes a phosphate group (5'-phosphate group); and
a second ligase, wherein the second ligase is capable of ligating the second modifying group at the 5' end of the second oligonucleotide of the 3' end adapter to a 3'-hydroxyl end (3'-OH) of the oligonucleotide (i.e., ligating the 5'-phosphate group of the second modifying group to the 3'OH), that is, the second ligase possesses the ligase activity of ligating the 5'-phosphate group of the second modifying group to the 3' OH.

The adapter composition can ligate the 5' end adapter to the 5' end of a single-stranded template nucleic acid through the first oligonucleotide of the 5' end adapter and the first ligase, and ligate the 3' end adapter to the 3' end of the single-stranded template nucleic acid through the second oligonucleotide of the 3' end adapter and the second ligase, such that both the 5' end adapter and the 3' end adapter can be ligated respectively to the 5' end and 3' end of the single-stranded template nucleic acid in the same reaction system simultaneously without the need to add the 5' end adapter and 3' end adapter in separate steps. This greatly reduces the reaction steps and library construction time, increases the efficiency of adapter ligation, ensures the directionality of adapter ligation, reduces the loss of template nucleic acid, and improves the utilization rate of the original template nucleic acid. **In** addition, no additional fixed sequence needs to be added to the template, the sequencing quality will not be impaired, and there is no need to perform additional truncation by a certain length of the sequenced reads. Furthermore, the adapters are prevented from ligating with each other at all.

Preferably, the phosphate group in the first modifying group is an artificially added modification.

Preferably, the phosphate group in the second modifying group is inherent in the second oligonucleotide.

Preferably, the first modifying group is at least one modification selected from the group consisting of phosphorylation modification, 2'3' cyclic phosphate modification, and pre-guanosine modification, and the like.

Preferably, the second modifying group is at least one modification selected from the group consisting of phosphorylation modification, and adenylation modification.

Preferably, the 5' end adapter may further include a third oligonucleotide, wherein the third oligonucleotide is in complementary with the first oligonucleotide to form a double strand, and a length of the third oligonucleotide is greater than, equal to or less than a length of the first oligonucleotide. More preferably, the length of the third oligonucleotide is less than the length of the first oligonucleotide, so that the 5' end adapter is a partially double-stranded adapter, and the 3' end of the partially double-stranded adapter has an overhang structure with several bases (which refers to a structure composed of two complementary nucleic acids of unequal lengths, where the 3' end of the short nucleic acid is aligned with the 5' end of the long nucleic acid, and the 3' end of the long nucleic acid exceeds the 5' end of the short nucleic acid, i.e., the overhang bases at the 3' end of the long nucleic acid are in a single-stranded state); that is, the 5' end adapter may be a single-stranded adapter (without the third oligonucleotide) or a partially double-stranded adapter (with the third oligonucleotide, where the 3' end of the partially double-stranded adapter has at least an overhang structure of several bases, preferably two bases).

Preferably, the difference between the base numbers of the third oligonucleotide and the first oligonucleotide is greater than or equal to 2.

Preferably, the third oligonucleotide has a third blocking group at the 5' end, and the third oligonucleotide has a fourth blocking group at the 3' end.

Preferably, the 3' end adapter may further include a fourth oligonucleotide, wherein the fourth oligonucleotide is in complementary with the second oligonucleotide to form a double strand; that is, the 3' end adapter may be a single-stranded adapter (without the fourth oligonucleotide) or a partially complementary double-stranded adapter (with the fourth oligonucleotide).

Preferably, a length of the fourth oligonucleotide is greater than, equal to or less than a length of the second oligonucleotide.

Preferably, the fourth oligonucleotide has a fifth blocking group at the 5' end, and the fourth oligonucleotide has a sixth blocking group at the 3' end.

Preferably, the first ligase is a type of enzyme that is capable of ligating the 3' phosphate end of an oligonucleotide to the 5' hydroxyl end of another oligonucleotide, and may be at least one selected from the group consisting of a RtcB ligase, an active fragment of RtcB ligase, a derivative of RtcB ligase, or an analog of RtcB ligase. Further, the first ligase may include RtcB ligase. The RtcB ligase may be one from any species. "An active fragment of RtcB ligase, a derivative of RtcB ligase, or an analog of RtcB ligase" refer to polypeptides that essentially maintain the same biological function or activity of the RtcB ligase according to the present disclosure. The derivative or analog according to the present disclosure may be (i) a polypeptide in which one or more (for example, 1-50, 1-40, 1-30, 1-20, 1-10, 1-5, 1-3, 1-2) conservative or non-conservative amino acid residues (preferably conservative amino acid residues) are substituted, and the substituent amino acid residue may or may not be encoded by genetic codes, or (ii) a polypeptide with a substituent group in one or more (for example, 1-50, 1-40, 1-30, 1-20, 1-10, 1-5, 1-3, 1-2) amino acid residues, or (iii) a polypeptide formed by fusion of an additional amino acid sequence to the polypeptide sequence (for example, a leader sequence, or a secretory sequence, or a sequence for purifying the polypeptide, or a proprotein sequence, or a fusion protein). According to the definitions herein, these active fragment, derivative, and analog belong to the scope known to those skilled in the art.

In the present disclosure, the active fragment of RtcB ligase refers to a polypeptide that still maintain all or part of the function of the full-length RtcB ligase. Generally, the active fragment may maintain at least 50% of the activity of the full-length RtcB ligase. In more preferred conditions, the active fragment may maintain 60%, 70%, 80%, 90%, 95%, 99%, or 100% of the activity of the full-length RtcB ligase.

In the present disclosure, the RtcB ligase may further include a variant form having the same function as RtcB ligase. The variant form may include (but is not limited to): deletion, insertion and/or substitution of several (typically 1-50, preferably 1-30, more preferably 1-20, optimally 1-10, and still more preferably 1-8, 1-5) amino acids, and addition or deletion of one or several (typically 1-50, preferably 1-30, more preferably 1-20, optimally 1-10, and still more preferably 1-8, 1-5) amino acids at the C-terminus and/or N-terminus (especially the N-terminus). For example, in the art, when substituting with amino acids of similar or closely related properties, the function of the protein may generally not be changed. For another example, adding or deleting one or several amino acids at the C-terminus and/or N-terminus (especially the N-terminus) may generally not change the function of the protein.

Any protein with high homology to the RtcB ligase (e.g., homology of 60% or higher, 70% or higher, 80% or higher with the sequence of the RtcB ligase; preferably, homology of 85% or higher; more preferably, homology of 90% or higher, for example, homology of 95%, 98% or 99%) and having the same function as the RtcB ligase is also included in the present disclosure. The term "homology" refers to the level of similarity (i.e., sequence similarity or identity) between two or more nucleic acids or polypeptides with reference to the percentage of identical positions. In the present disclosure, the variants of gene can be obtained by inserting or deleting the regulatory region, performing random or site-directed mutation, etc.

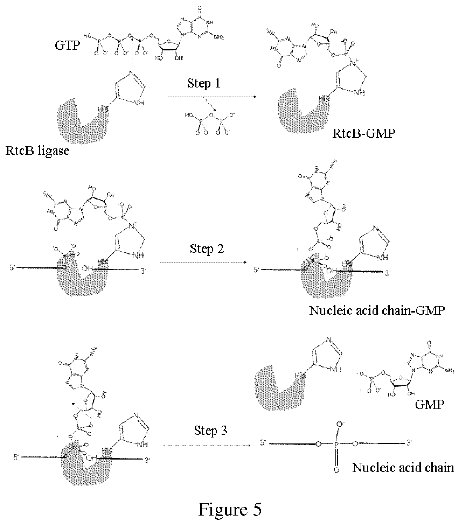

The principle ligation of RtcB ligase (the first ligase) is as shown in Figure 5, specifically as follows: (1) In the presence of Mn ions, the RtcB ligase (the first ligase) reacts with a triphosphate (e.g., GTP) to generate a covalent RtcB-histidine-GMP intermediate and releases a pyrophosphate (Ppi); (2) The GMP is then transferred to a 3' phosphate group or 2'3' cyclic phosphate group at the end of a nucleic acid chain; (3) Afterwards, another nucleic acid chain with a 5'-OH attacks the activated 3'-P nucleic acid chain to form a 3', 5'-phosphodiester bond and release the GMP. Therefore, any enzyme that is capable of completing the nucleic acid ligation according to the above process falls within the protection scope of the first ligase described in the present disclosure.

Preferably, the first ligase may include thermophilic RNA ligase RtcB.

Preferably, the adapter composition may further include a first cofactor, and the first cofactor may include a triphosphate and a manganese ion, which are used for assisting the first ligase in ligating the first modifying group at the 3' end of the first oligonucleotide of the 5' end adapter to the 5'-hydroxyl end of the template nucleic acid.

The first cofactor is used for the first ligase (e.g., a RtcB ligase) in catalyzing the ligation reaction between an oligonucleotide with a 3' end phosphorylation modification (including 3' phosphate group or 2'3' cyclic phosphate group) (3'P oligonucleotide) and another oligonucleotide with 5' end hydroxyl modification (5' OH oligonucleotide) through a three-step nucleic acid transfer process: (1) RtcB ligase reacts with a triphosphate (e.g. GTP) to generate a covalent RtcB-histidine-GMP intermediate and release a PPi; (2) The GMP is transferred to the 3' phosphate or 2'3' cyclic phosphate group of an oligonucleotide; (3) another 5' OH oligonucleotide attacks the activated 3'P oligonucleotide to form a 3', 5'-phosphodiester bond and release the GMP.

Preferably, the triphosphate includes at least one of NTP and dNTP; and further, the triphosphate includes at least one of ATP, CTP, GTP, TTP, UTP, dATP, dCTP, and dGTP.

Preferably, the manganese ion is a divalent manganese ion.

Preferably, the adapter composition may further include a second cofactor, and the second cofactor may include at least one of ATP and NAD, and a metal ion, which is used for assisting the second ligase in ligating the second modifying group at the 5' end of the second oligonucleotide of the 3' end adapter to the 3'-hydroxyl end of the template nucleic acid.

Preferably, the metal ion includes at least one of magnesium ion and manganese ion.

Preferably, the manganese ion is a divalent manganese ion.

Preferably, when the 5' end of the second oligonucleotide of the 3' end adapter is phosphorylated (i.e., has a phosphorylation modification), the second ligase includes at least one of T4 RNA ligase 1, T4 DNA ligase, TS2126 RNA ligase, T3 DNA ligase, E. coli DNA ligase, single-stranded DNA/RNA circligase (ssDNA/RNA CircLigase), T4 RNA ligase 2, and Taq DNA ligase; and further, the second ligase includes T4 RNA ligase 1.

Preferably, when the 5' end of the second oligonucleotide of the 3' end adapter is adenylated (i.e., has an adenylation modification), the second ligase includes at least one of T4 RNA ligase 1, T4 RNA ligase 2, T4 RNA ligase 2 (truncated K227Q), T4 RNA ligase 2 (truncated KQ), thermostable 5' App DNA/RNA ligase, TS2126 RNA ligase, T3 DNA ligase, E. coli DNA ligase, single-stranded DNA/RNA circligase (ssDNA/RNA CircLigase), and Taq DNA ligase.

Preferably, the adapter composition may further include a reaction buffer, which is used for the adapter ligation reaction catalyzed by the first ligase and/or the second ligase.

Preferably, the first blocking group is a modification selected from the group consisting of amino modification, dideoxy modification, spacer modification, and phosphate modification.

Preferably, the second blocking group is a modification selected from the group consisting of amino modification, dideoxy modification, spacer modification, and phosphate modification.

Preferably, the third blocking group is a modification selected from the group consisting of amino modification, dideoxy modification, spacer modification, and phosphate modification.

Preferably, the fourth blocking group is a modification selected from the group consisting of amino modification, dideoxy modification, spacer modification, and phosphate modification.

Preferably, the fifth blocking group is a modification selected from the group consisting of amino modification, dideoxy modification, spacer modification, and phosphate modification.

Preferably, the sixth blocking group is a modification selected from the group consisting of amino modification, dideoxy modification, spacer modification, and phosphate modification.

By means of the modification of the first blocking group, the second blocking group, the third blocking group, the fourth blocking group, the fifth blocking group and the sixth blocking group, the adapters are prevented from ligating with each other.

Preferably, the modification of the first blocking group and the second blocking group are amino modification.

Preferably, the amino modification includes at least one of C6 amino modification and C12 amino modification; and further includes C6 amino modification.

Preferably, the sequence of the 5' end adapter is as shown in SEQ ID NO. 1, with C6 amino modification at the 5' end and phosphorylation modification at the 3' end.

Preferably, the sequence of the 3' end adapter is as shown in SEQ ID NO. 2, with phosphorylation modification at the 5' end and C6 amino modification at the 3' end.

Preferably, the first oligonucleotide of the 5' end adapter and/or the second oligonucleotide of the 3' end adapter includes an adapter sequence.

Preferably, the first oligonucleotide of the 5' end adapter and/or the second oligonucleotide of the 3' end adapter may further include a sequencing primer sequence.

Preferably, the first oligonucleotide of the 5' end adapter and/or the second oligonucleotide of the 3' end adapter may further include a sample tag sequence, which is used for distinguishing different samples for subsequent multi-sample mixed sequencing. For example, the sample tag sequence may be a barcode sequence or an index sequence.

Preferably, the first oligonucleotide of the 5' end adapter and/or the second oligonucleotide of the 3' end adapter may further includes a unique molecular identifier (UMI) sequence, which is used for counting the copies of nucleic acid molecules in the sample.

Preferably, the first oligonucleotide of the 5' end adapter includes an adapter sequence.

Preferably, the first oligonucleotide of the 5' end adapter may further include a sequencing primer sequence.

Preferably, the first oligonucleotide of the 5' end adapter may further include a sample tag sequence, which is used for distinguishing different samples for subsequent multi-sample mixed sequencing. For example, the sample tag sequence may be a barcode sequence or an index sequence.

Preferably, the first oligonucleotide of the 5' end adapter may further include a unique molecular identifier (UMI) sequence, which is used for counting the copies of nucleic acid molecules in the sample.

Preferably, the second oligonucleotide of the 3' end adapter includes an adapter sequence.

Preferably, the adapter sequence of the first oligonucleotide of the 5' end adapter may be the same as or different from the adapter sequence of the second oligonucleotide of the 3' end adapter.

Preferably, the second oligonucleotide of the 3' end adapter may further include a sequencing primer sequence.

Preferably, the sequencing primer sequence of the first oligonucleotide of the 5' end adapter may be the same as or different from the sequencing primer sequence of the second oligonucleotide of the 3' end adapter.

Preferably, the second oligonucleotide of the 3' end adapter may further include a sample tag sequence, which is used for distinguishing different samples for subsequent multi-sample mixed sequencing. For example, the sample tag sequence may be a barcode sequence or an index sequence.

Preferably, the sample tag sequence of the first oligonucleotide of the 5' end adapter may be the same as or different from the sample tag sequence of the second oligonucleotide of the 3' end adapter.

Preferably, the second oligonucleotide of the 3' end adapter may further include a unique molecular identifier (UMI) sequence, which is used for counting the copies of nucleic acid molecules in the sample.

Preferably, the UMI sequence of the first oligonucleotide of the 5' end adapter may be the same as or different from the UMI sequence of the second oligonucleotide of the 3' end adapter.

Preferably, a length of the sample tag sequence is 5 to 20 bp.

Preferably, a length of the unique molecular identifier is 5 to 20 bp.

Preferably, the first oligonucleotide of the 5' end adapter is different from the second oligonucleotide of the 3' end adapter.

The sequencing primer sequence, the sample tag sequence, and the unique molecular index (UMI) sequence of the first oligonucleotide of the 5' end adapter and/or the second oligonucleotide of the 3' end adapter may also be introduced by PCR amplification or reverse transcription reaction after the ligation of the single-stranded template nucleic acid to the adapter; that is, they may be introduced into the PCR primer or the reverse transcription primer.

Preferably, the adapter composition may further include a phosphatase, which is used for removing the phosphate group at the 5' end and/or 3' end of the single-stranded template nucleic acid or the double-stranded nucleic acid containing the single-stranded template nucleic acid, thereby ensuring that the 5' end and 3' end of the single-stranded template nucleic acid or the double-stranded nucleic acid containing the single-stranded template nucleic acid are both hydroxyl groups, preventing self-ligation under the action of ligase.

Preferably, the phosphatase includes: at least one of acid phosphatase and alkaline phosphatase; further, the phosphatase includes: alkaline phosphatase; further, the phosphatase includes: at least one of calf intestinal phosphatase, shrimp alkaline phosphatase, Antarctic phosphatase and APEX alkaline phosphatase; and further, the phosphatase includes: shrimp alkaline phosphatase.

Preferably, the adapter composition is used for ligating the 5' end adapter and 3' end adapter respectively to the 5' end and 3' end of the single-stranded template nucleic acid.

Preferably, the adapter composition is used for ligating the 5' end adapter and the 3' end adapter respectively to the 5' end and 3' end of the single-stranded template nucleic acid in the same system; wherein the same system specifically refers to carrying out the reaction in the same reaction vessel; and further specifically, to carrying out the reaction in the same reaction vessel at the same time.

Preferably, the single-stranded template nucleic acid is obtained by subjecting the nucleic acid to at least one treatment of fragment, denaturation, and DNA methylation.

Preferably, the nucleic acid includes at least one of DNA and RNA.

Preferably, the DNA includes at least one of dsDNA and ssDNA.

Preferably, the nucleic acid includes dsDNA, ssDNA and RNA.

Preferably, the nucleic acid is derived from the following biological samples: cells, fresh tissues, fresh organs, decayed tissues, formalin-fixed tissues, paraffin-embedded tissues, forensic samples, paleontological fossils, or biological materials containing cfDNA or RNA.

Preferably, the biological materials include but are not limited to: peripheral blood, plasma, serum, urine, feces, saliva, cerebrospinal fluid, lymph fluid, bronchoalveolar lavage fluid, amniotic fluid, blastocyst cavity fluid, cell culture fluid, embryo culture fluid, microbial culture medium, soil leachate, or bone meal leachate.

More specifically, the nucleic acid may be isolated from a biological sample obtained from an individual (e.g., a test individual). The individual may be any living or non-living organism, including but not limited to humans, non-human animals, plants, bacteria, fungi, protists, or pathogens.

The nucleic acid may be isolated or obtained from any suitable type of biological samples. The nucleic acid may be isolated or obtained from a single cell, multiple cells (e.g., cultured cells), cell culture media, conditioned media, tissues, organs, or organisms (e.g., bacteria, yeast, etc.).

**In** some cases, the nucleic acid as a part of forensic analysis may be obtained. **In** some embodiments, the kits described herein may be applied onto forensic samples or specimens. Forensic samples or specimens may include any biological material containing nucleic acids. For example, the forensic samples or specimens may include blood, semen, hair, skin, sweat, saliva, decomposed tissue, bone, nail scrapings, licked stamp/envelope, sluff, touch DNA, razor residues, etc. The specimens may be formalin-fixed tissues and/or paraffin-embedded tissues.

The biological sample may be any sample isolated or obtained from an individual or a portion thereof (such as human individuals, pregnant females, cancer patients, patients with infections or infectious diseases, transplant recipients, fetuses, tumors, infected organs or tissues, transplanted organs or tissues, or microbiomes). **In** some embodiments, the biological sample is obtained from an individual's cervical swab. The liquid sample or tissue sample from which nucleic acids are extracted may be acellular (for example, cell-free). **In** some embodiments, the biological sample may contain cellular components or cellular remnants. **In** some embodiments, the biological sample may include fetal cells or cancer cells.

The biological sample may be a liquid sample. The liquid sample may contain extracellular nucleic acids (e.g. circulating cell-free DNA). Examples of liquid sample include but are not limited to blood or blood products (e.g., serum, plasma, etc.), urine, cerebrospinal fluid, saliva, sputum, biopsy samples (e.g., liquid biopsies for cancer detection), analogues or combinations thereof the aforementioned liquid samples. In some embodiments, the biological sample is liquid biopsies, which typically refer to the liquid sample from an individual for assessment of the presence, absence, progression, or remission of diseases (e.g., cancer). The liquid biopsy may be used in conjunction with or as an alternative to commercial biopsies (e.g., tumor biopsies). In some cases, the extracellular nucleic acids are analyzed in a liquid biopsy.

The biological sample may be a tumor nucleic acid sample (i.e. a nucleic acid sample isolated from tumor).

Preferably, when the nucleic acid contains dsDNA, a process of preparing the single-stranded template nucleic acid may include denaturation treatment.

Preferably, when the nucleic acid is derived from the following biological samples: cells, fresh tissues, fresh organs, paraffin-embedded tissues, or forensic samples, the process of preparing the single-stranded template nucleic acid may include fragment treatment.

Preferably, when the nucleic acid is used for constructing a DNA methylation library, the process of preparing the single-stranded template nucleic acid may include DNA methylation treatment.

Preferably, the denaturation treatment method includes: thermal denaturation and/or chemical denaturation.

More specifically, double-stranded nucleic acids (e.g., dsDNA) may be chemically denatured using a denaturant (for example, alkali, glycerol, ethylene glycol, formamide, urea or a combination thereof) that reduces the melting temperature of double-stranded nucleic acids. The melting temperature may be reduced by 5°C-6°C by adding every 10% (v/v) of the denaturant to the reaction mixture. The denaturant or a combination of denaturants (e.g., 10% glycerol and 6-7% ethylene glycol) may account for 1%, 5%, 10%, 15%, 20%, or 25% (v/v) of the reaction mixture. Salts that reduce hybridization stringency may be included in the reaction buffer at low concentration to chemically denature double-stranded nucleic acids at low temperature. Double-stranded nucleic acids (e.g., dsDNA) may be denatured by heating, for example, at 92-98 °C for 3-10 minutes. The denatured double-stranded nucleic acid may be placed on ice immediately after denaturation to prevent renaturation.

Preferably, the method of fragment treatment includes at least one of a chemical fragment method and a physical fragment method.

Preferably, the method of DNA methylation treatment involves treatment with a DNA methylation modification conversion reagent.

Preferably, the DNA methylation modification conversion reagent is at least one selected from the group consisting of bisulfite, sulfite, metabisulfite, and metabisulfite.

Preferably, the adapter composition may further includes a substance for nucleic acid fragment.

Preferably, the substance for nucleic acid fragment is one or more fragment methods selected from the group consisting of a chemical fragment method and a physical fragment method.

Preferably, the chemical fragment method includes at least one of a transposase method and a conventional enzymatic fragmentation method.

Preferably, the physical fragment method includes at least one of an ultrasonic fragment method and a mechanical fragment method.

Preferably, the adapter composition may further include a substance for nucleic acid denaturation.

Preferably, the substance for nucleic acid denaturation is a substance for one or more denaturation methods of thermal denaturation and chemical denaturation.

Preferably, when the adapter composition is used for constructing a DNA methylation library, the adapter composition may further include a DNA methylation modification conversion reagent, which is capable of converting non-methylated C bases in DNA into U bases.

Preferably, the DNA methylation modification conversion reagent is at least one selected from the group consisting of bisulfite, sulfite, metabisulfite, and metabisulfite.

Preferably, the adapter composition may further include a single-strand binding protein (SSB), which is used for maintaining the single-stranded state of single-stranded nucleic acids.

Single-strand binding protein refers to any protein that has the function of binding to a single-stranded nucleic acid, for example, for preventing premature annealing, protecting single-stranded nucleic acids from nuclease digestion, removing secondary structures from nucleic acids, or facilitating the replication of nucleic acids. The term is intended to include, but is not necessarily limited to, the proteins formally identified as single-strand binding protein by the Nomenclature Committee of the International Union of Biochemistry and Molecular Biology (NC-IUBMB). Exemplary single-strand binding proteins include, but are not limited to, *Escherichia coli* (E.coli) SSB, T4gp32, T7 gene 2.5SSB, bacteriophage π29SSB, MjA SSB from *Methanococcus jannaschii,* any homologous protein from any phylum, or protein complex, or functional variants thereof.

Preferably, the first oligonucleotide of the 5' end adapter and/or the second oligonucleotide of the 3' end adapter is immobilized on a solid phase support. More preferably, a non-ligating end of the first oligonucleotide of the 5' end adapter and/or the second oligonucleotide of the 3' end adapter is immobilized on a solid phase support. For the first oligonucleotide of the 5' end adapter, the ligating end thereof is the 3' end of the first oligonucleotide of the 5' end adapter; and for the second oligonucleotide of the 3' end adapter, the ligating end thereof is the 5' end of the second oligonucleotide of the 3' end adapter. Through the ligation reaction with the 5' end adapter and/or the 3' end adapter immobilized on the solid phase support, one or more single-stranded template nucleic acids can be attached to the solid phase support, thereby generating a solid phase support with a specific sequence (adapter) and the single-stranded template nucleic acid, which can be used for single-cell sequencing, spatial omics sequencing, etc.

Preferably, the solid phase support is selected from the group consisting of a chip and a bead, and the like.

Preferably, the chip may be one that is usable in a sequencing platform, for example, a sequencing chip.

Preferably, the chip is a high-throughput sequencing chip, for example, a high-throughput sequencing chip for Illumina, MGI or Thermo Fisher sequencing platform.

Preferably, the first oligonucleotide of the 5' end adapter and/or the second oligonucleotide of the 3' end adapter may be immobilized on a solid phase support (chip) by any suitable method known in the art.

Preferably, non-limiting examples of the method include nucleic acid hybridization, biotin-streptavidin binding, thiol binding, photoactivation binding, covalent binding, antibody-antigen binding, physical constraint through hydrogel or other porous polymers, or any combination thereof.

Preferably, the chip includes a material selected from the group consisting of glass, silicon, polylysine coating material, nitrocellulose, and polystyrene, cyclic olefin copolymers (COCs), cyclic olefin polymers (COPs), polypropylene, polyethylene, polycarbonate, or any combination thereof.

Preferably, the bead is selected from the group consisting of agarose gel beads, agarose beads, magnetic beads, protein A conjugated beads, protein G conjugated beads, protein L conjugated beads, oligo (dT) conjugated beads, silica beads, hydrogel beads, silica-like beads, anti-biotin microbeads, anti-fluorescent dye microbeads, and any combination thereof.

Preferably, the first oligonucleotide of the 5' end adapter and/or the second oligonucleotide of the 3' end adapter may be immobilized on the solid phase support (bead) by any suitable method known in the art, for example, 1) or 2):
1) The solid phase support (bead) and the nucleic acid (the first oligonucleotide of the 5' end adapter and/or the second oligonucleotide of the 3' end adapter) are respectively modified with functional units that can interact with each other, so that they react to label the nucleic acid on the solid phase support; and
2) The nucleic acid (the first oligonucleotide of the 5' end adapter and/or the second oligonucleotide of the 3' end adapter) is directly synthesized on the solid phase support (bead) according to the preset nucleotide sequence.

More specifically, the first oligonucleotide of the 5' end adapter and/or the second oligonucleotide of the 3' end adapter may be attached to the solid phase support (bead) using "graft to" and/or "graft from" approaches. **In** specific applications, the solid phase support (bead) may be labeled with nucleic acid (the first oligonucleotide of the 5' end adapter and/or the second oligonucleotide of the 3' end adapter) using the "graft to" approach alone. **In** specific applications, the solid phase support (bead) may be labeled with nucleic acid (the first oligonucleotide of the 5' end adapter and/or the second oligonucleotide of the 3' end adapter) using the "graft from" approach alone. **In** specific applications, the solid phase support (bead) may be labeled with nucleic acid (the first oligonucleotide of the 5' end adapter and/or the second oligonucleotide of the 3' end adapter) using a combination of "graft to" and "graft from" approaches.

Preferably, the bead includes a material selected from the group consisting of polydimethylsiloxane (PDMS), polystyrene, glass, polypropylene, agarose, gelatin, hydrogel, paramagnetic material, ceramic, plastic, glass, methylstyrene, acrylic polymer, titanium, latex, agarose gel, cellulose, nylon, silicone, or any combination thereof.

Preferably, when the "graft to" approach is used, the solid phase support and the nucleic acid are respectively modified with functional units that can interact with each other, and the functional units include but are not limited to one or more of hydroxyl, aldehyde, epoxy, amino, carboxyl and a activated form thereof, phosphate, alkynyl, azide, thiol, alkene, biotin, avidin, isothiocyanate, isocyanate, acyl azide, sulfonyl chloride, toluenesulfonyl ester, and the like.

Preferably, when the "graft from" approach is used, the preset nucleotide sequence are directly synthesized on the support.

According to second aspect of the present disclosure, provided is a kit for nucleic acid library construction, which includes: the adapter composition of the first aspect of the present disclosure.

Preferably, the kit for nucleic acid library construction may further include a polynucleotide kinase (preferably T4 polynucleotide kinase), which is used for dephosphorylation of the 3' end of the single-stranded template nucleic acid ligated with adapters. Preferably, the 5' end adapter does not include a third oligonucleotide, the 3' end adapter does not include a fourth oligonucleotide, and the modification of the first blocking group and the modification of the second blocking group are phosphate modification. After the 3'-end dephosphorylation, the single-stranded template nucleic acid ligated with adapters is then subjected to a cyclization reaction to obtain a single-strand cyclization library, thereby achieving the PCR-free nucleic acid library construction.

Preferably, the kit for nucleic acid library construction further include a PCR primer, which is used for PCR amplification of the single-stranded template nucleic acid ligated with adapters.

Preferably, the PCR primer contains a sequence that is partially or fully identical or complementary to the adapter.

Preferably, the adapter includes a 3' end adapter and/or a 5' end adapter.

Preferably, when the adapter does not include a sequencing primer sequence, a sequencing primer sequence may be introduced by a PCR primer, and the PCR primer may further include the sequencing primer sequence.

Preferably, when the adapter does not include a sample tag sequence, a sample tag sequence may be introduced by a PCR primer, and the PCR primer may further include the sample tag sequence, which is used for distinguishing different samples for subsequent multi-sample mixed sequencing. For example, the sample tag sequence may be a barcode sequence or an index sequence.

Preferably, when the adapter does not include a unique molecular identifier (UMI) sequence, a unique molecular identifier sequence may be introduced by a PCR primer, and the PCR primer may further include the unique molecular identifier (UMI) sequence, which is used for counting the copies of nucleic acid molecules in the sample.

Preferably, when the adapter does not include a sequencing primer sequence, a sample tag sequence, or a unique molecular identifier (UMI) sequence, the PCR primer may include a first primer and a second primer;
wherein, the first primer contains a sequence that is partially or fully identical or complementary to the 5' end of the first oligonucleotide of the 5' end adapter, and the second primer contains a sequencing primer sequence, a sample tag sequence, and a sequence that is partially or fully identical or complementary to the 3' end of the second oligonucleotide of the 3' end adapter; and preferably, the second primer may further contain a unique molecular identifier sequence; or
the first primer contains a sequencing primer sequence, a sample tag sequence, and a sequence that is partially or fully identical or complementary to the 5' end of the first oligonucleotide of the 5' end adapter, and the second primer contains a sequence that is partially or fully identical or complementary to the 3' end of the second oligonucleotide of the 3' end adapter; and preferably, the first primer may further contain a unique molecular identifier sequence.

Preferably, the sequences of the PCR primer are as shown in SEQ ID NOs. 3 and 4.

Preferably, the kit for nucleic acid library construction may further include a PCR reaction mixture.

Preferably, when the nucleic acid contains RNA, the kit for nucleic acid library construction may further include a reverse transcription primer, which is used for reverse transcription of RNA to obtain cDNA.

Preferably, the reverse transcription primer includes a sequence that is partially or fully identical or complementary to the 3' end of the second oligonucleotide of the 3' end adapter.

Preferably, when the adapter does not include a sequencing primer sequence, a sequencing primer sequence may be introduced through a reverse transcription primer, and the reverse transcription primer may further include a sequencing primer sequence.

Preferably, when the adapter does not include a sample tag sequence, a sample tag sequence may be introduced through a reverse transcription primer, and the reverse transcription primer may further include a sample tag sequence, which is used for distinguishing different samples for subsequent multi-sample mixed sequencing. For example, the sample tag sequence may be a barcode sequence or an index sequence.

Preferably, when the adapter does not include a unique molecular identifier (UMI) sequence, a unique molecular identifier sequence may be introduced by a reverse transcription primer, and the reverse transcription primer may further include a unique molecular identifier (UMI) sequence, which is used for counting the copies of nucleic acid molecules in the sample.

Preferably, when the nucleic acid is RNA, the kit for nucleic acid library construction may further include a reverse transcription reaction solution.

Preferably, the kit for nucleic acid library construction may further include a nucleic acid extraction reagent, wherein the nucleic acid extraction reagent is a lysis reagent.

Preferably, the kit for nucleic acid library construction may further include a nucleic acid extraction reagent combination, specifically a nucleic acid extraction reagent combination for an extraction method selected from the group consisting of alkaline lysis method, phenolchloroform extraction method, chelating resin method, spin column membrane adsorption method, and magnetic beads method.

Preferably, the nucleic acid extraction reagent combination includes: at least one of a lysis solution, a washing solution, an elution solution, and a nucleic acid adsorbing material. More preferably, the nucleic acid extraction reagent combination includes: a lysis solution, a washing solution, an elution solution, and a nucleic acid adsorbing material.

Preferably, the nucleic acid adsorbing material includes at least one of magnetic bead and adsorption membrane.

Preferably, the kit for nucleic acid library construction may further include a cyclization reagent combination.

According to a third aspect of the present disclosure, provided is a sequencing reagent kit, which includes: any one of a1) and a2):
a1) the adapter composition of the first aspect of the present disclosure; and
a2) the kit for nucleic acid library construction of the second aspect of the present disclosure.

Preferably, the sequencing reagent kit may further include a sequencing kit.

According to a fourth aspect of the present disclosure, provided is a sequencing system, which includes: any one of a1) to a3), and a sequencer:
a1) the adapter composition of the first aspect of the present disclosure;
a2) the kit for nucleic acid library construction of the second aspect of the present disclosure; and
a3) the sequencing reagent kit of the third aspect of the present disclosure.

According to a fifth aspect of the present disclosure, provided is a method for adapter ligation, which includes the step of using the adapter composition of the first aspect of the present disclosure.

Preferably, the method for adapter ligation includes the following steps: obtaining a single-stranded template nucleic acid, wherein the 5' end and the 3' end of the single-stranded template nucleic acid are both hydroxyl groups; and performing an adapter ligation reaction of a 5' end adapter and a 3' end adapter to the single-stranded template nucleic acid using the first ligase, the 5' end adapter, the second ligase and the 3' end adapter in the adapter composition of the first aspect of the present disclosure.

Preferably, the adapter ligation reaction is performed in the same system.

Preferably, the same system specifically refers to carrying out the reaction in the same reaction vessel; and further specifically, to carrying out the reaction in the same reaction vessel at the same time.

Preferably, the adapter ligation reaction is performed at a temperature of 15-82 °C; and further, 16-80 °C.

Preferably, the adapter ligation reaction is performed for more than 15 minutes; and further, 30-60 minutes.

Preferably, the adapter ligation is directional, and the first oligonucleotide of the 5' end adapter has a different nucleotide sequence from the second oligonucleotide of the 3' end adapter.

Preferably, the adapter ligation reaction system may further include the first cofactor in the adapter composition of the first aspect of the present disclosure.

Preferably, the adapter ligation reaction system may further include the second cofactor in the adapter composition of the first aspect of the present disclosure.

Preferably, the adapter ligation reaction system may further include the reaction buffer in the adapter composition of the first aspect of the present disclosure.

Preferably, the single-stranded template nucleic acid with hydroxyl groups at both the 5' end and the 3' end is obtained by dephosphorylation treatment, including the following steps: treating a single-stranded template nucleic acid or a double-stranded nucleic acid containing the single-stranded template nucleic acid with a phosphatase, thereby removing the phosphate groups at the 5' end and/or the 3' end of the single-stranded template nucleic acid or the double-stranded nucleic acid containing the single-stranded template nucleic acid.

Preferably, the single-stranded template nucleic acid is obtained by subjecting the nucleic acid to at least one treatment of fragment, denaturation, and DNA methylation.

Preferably, the nucleic acid includes at least one of DNA and RNA.

Preferably, the DNA includes at least one of dsDNA and ssDNA.

Preferably, the nucleic acid includes at least one of dsDNA, ssDNA, and RNA.

Preferably, the nucleic acid is derived from the following biological samples: cells, fresh tissues, fresh organs, decayed tissues, formalin-fixed tissues, paraffin-embedded tissues, forensic samples, paleontological fossils, or biological materials containing cfDNA or cfRNA.

Preferably, the biological materials include but are not limited to: peripheral blood, plasma, serum, urine, feces, saliva, cerebrospinal fluid, lymph fluid, bronchoalveolar lavage fluid, amniotic fluid, blastocyst cavity fluid, cell culture fluid, embryo culture fluid, microbial culture medium, soil leachate, or bone meal leachate.

Preferably, when the nucleic acid contains dsDNA, a process of preparing the single-stranded template nucleic acid may include denaturation treatment.

Preferably, the denaturation treatment may be performed before or after the phosphorylation treatment.

Preferably, the denaturation treatment method includes: thermal denaturation and/or chemical denaturation; more preferably, thermal denaturation; still more preferably, treatment at 92-98 °C for 3-10 minutes.

Preferably, the denaturation treatment may further include a step of preventing the renaturation of the denatured double-stranded nucleic acid: immediately placing on ice after the denaturation is completed.

Preferably, when the nucleic acid is derived from the following biological samples: cells, fresh tissues, fresh organs, paraffin-embedded tissues, or forensic samples, the process of preparing the single-stranded template nucleic acid may include fragment treatment.

Preferably, the fragment treatment is performed before the phosphorylation treatment and the denaturation treatment.

Preferably, the method of fragment treatment includes at least one of a chemical fragment method and a physical fragment method.

Preferably, the chemical fragment method includes at least one of a transposase method and a conventional enzymatic fragmentation method.

Preferably, the physical fragment method includes at least one of an ultrasonic fragment method and a mechanical fragment method.

Preferably, when the nucleic acid is used for constructing a DNA methylation library, the process of preparing the single-stranded template nucleic acid may include DNA methylation treatment.

Preferably, the DNA methylation treatment is performed before the phosphorylation treatment and the denaturation treatment.

Preferably, the DNA methylation treatment is performed after the fragment treatment.

Preferably, the DNA methylation treatment involves treatment with a DNA methylation modification conversion reagent.

Preferably, the DNA methylation modification conversion reagent is at least one selected from the group consisting of bisulfite, sulfite, metabisulfite, and metabisulfite.

According to a sixth aspect of the present disclosure, provided is a method for nucleic acid library construction, which includes the steps of the method for adapter ligation of the fifth aspect of the present disclosure.

Preferably, for a method for nucleic acid library construction using a PCR-free process, the method may further include the following steps: dephosphorylating the 3' end of the single-stranded template nucleic acid ligated with the adapter, and performing a cyclization reaction to obtain a single-strand cyclization library.

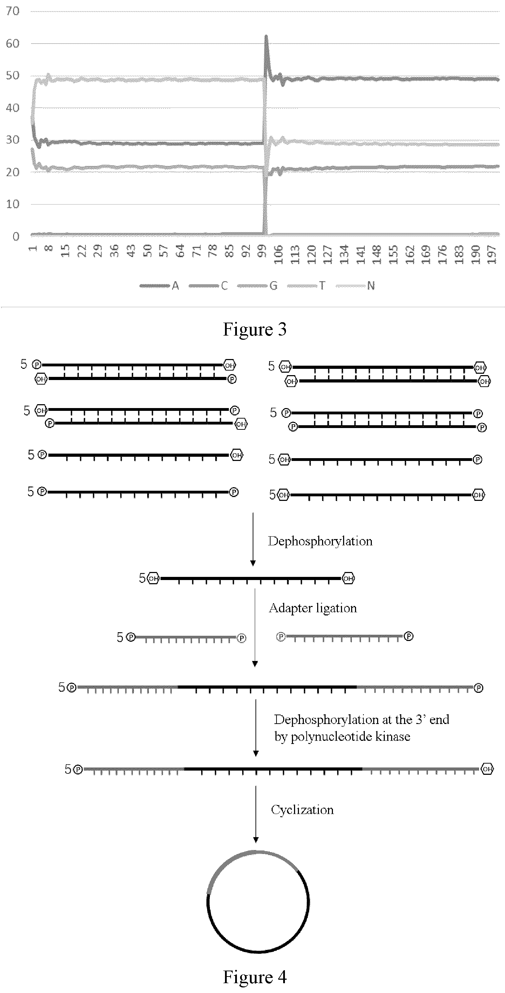

Preferably, as shown in Figure 4, the method for nucleic acid library construction includes the following steps:
obtaining a single-stranded template nucleic acid ligated with an adapter according to the method for adapter ligation of the fifth aspect of the present disclosure; and
dephosphorylating the 3' end of the single-stranded template nucleic acid ligated with the adapter, and performing a cyclization reaction to obtain a single-strand cyclization library.

Preferably, the 5' end adapter in the single-stranded template nucleic acid ligated with the adapter does not include a third oligonucleotide.

Preferably, the 3' end adapter in the single-stranded template nucleic acid ligated with the adapter does not include a fourth oligonucleotide.

Preferably, the modification of the first blocking group and the modification of the second blocking group in the single-stranded template nucleic acid ligated with the adapter are phosphate modifications.

Preferably, for a method for nucleic acid library construction needing a PCR process, the method may further include the step of performing an amplification reaction on the single-stranded template nucleic acid ligated with the adapter to obtain a nucleic acid library.

Preferably, the method for nucleic acid library construction includes the following steps:
obtaining a single-stranded template nucleic acid ligated with an adapter according to the method for adapter ligation of the fifth aspect of the present disclosure; and
performing an amplification reaction based on the single-stranded template nucleic acid ligated with the adapter to obtain a nucleic acid library.

Preferably, the amplification reaction is a rolling circle amplification or a linear amplification.

Preferably, the amplification reaction involves using the PCR primer in the kit for nucleic acid library construction of the second aspect of the present disclosure.

Preferably, when the nucleic acid contains RNA, the method for nucleic acid library construction may further include the step of performing a reverse transcription reaction; wherein, the reverse transcription reaction may be performed before the adapter ligation reaction or after the adapter ligation reaction.

Preferably, the reverse transcription reaction involves using the reverse transcription primer in the kit for nucleic acid library construction of the second aspect of the present disclosure.

Preferably, the method for nucleic acid library construction may further include the step of purifying the amplified product.

Preferably, the purification is performed using magnetic beads.

Preferably, the method for nucleic acid library construction may further include the step of performing a cyclization reaction after the adapter ligation reaction.

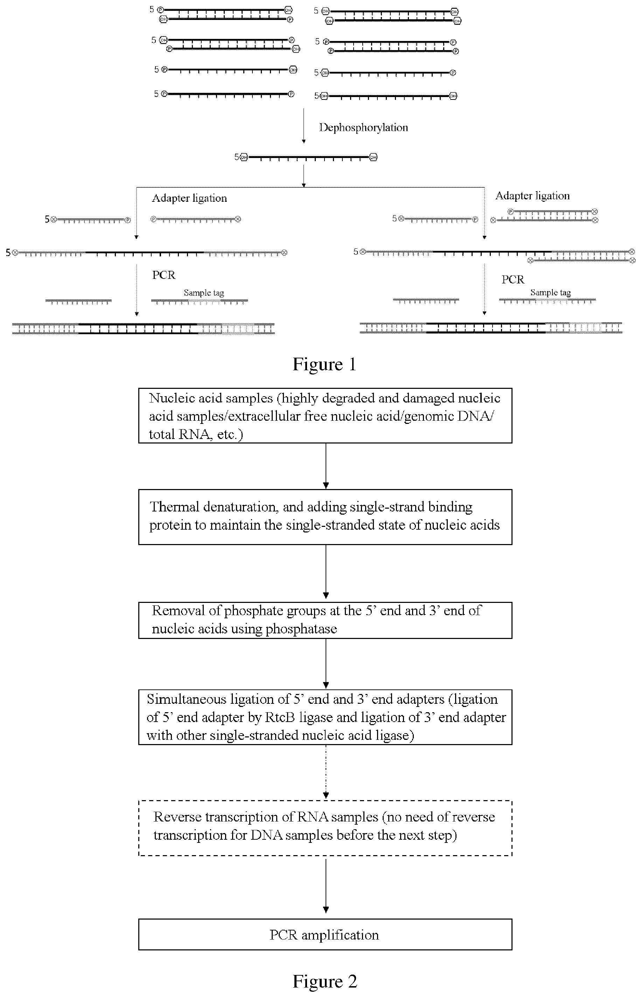

Preferably, as shown in Figures 1 and 2, a method for nucleic acid library construction includes the following steps:
(1) Obtaining a nucleic acid sample: the nucleic acid sample may be: 1) intact genomic DNA or RNA disrupted into fragments of a certain size; 2) extracted cfDNA or cfRNA; 3) degraded DNA or RNA extracted from various degraded biological samples, for example, paleontological fossils, and FFPE samples; or 4) DNA fragments disrupted from intact genomic DNA or DNA purified and recovered from extracellular free DNA after sulfite treatment.
(2) Denaturing the nucleic acid sample (this step is used for a nucleic acid sample containing double-stranded nucleic acid) to obtain a single-stranded template nucleic acid; at the same time, adding a single-strand binding protein to maintain the single-stranded state of the nucleic acid sample (single-stranded) or the single-stranded template nucleic acid using the property of the binding protein. This step can utilize both double-stranded nucleic acid and single-stranded nucleic acid in the sample.
(3) Dephosphorylating the single-stranded template nucleic acid, removing the phosphate groups at the 5' and 3' ends of the single-stranded template nucleic acid using a phosphatase, obtaining the single-stranded template nucleic acid with hydroxyl groups at both ends, thereby preventing the template nucleic acid from ligating to each other in the presence of ligase. This step may be performed after the step (1) and before the step (2);
(4) Ligating adapters: performing adapter ligation reaction on the single-stranded template nucleic acid using the first ligase, 5' end adapter, second ligase, and 3' end adapter in the adapter composition of the first aspect of the present disclosure; preferably, the adapter ligation reaction system may further include: the first cofactor and the second cofactor in the adapter composition of the first aspect of the present disclosure; and preferably, the adapter ligation reaction system may further include: the reaction buffer in the adapter composition of the first aspect of the present disclosure;
(5) Obtaining a library: performing a amplification reaction on the single-stranded template nucleic acid ligated with adapters to obtain a nucleic acid library; wherein, when the nucleic acid is RNA, a reverse transcription reaction is further included before the amplification reaction; preferably, the amplification reaction is performed using the PCR primer in the kit for nucleic acid library construction of the second aspect of the present disclosure; and preferably, the reverse transcription reaction is performed using the reverse transcription primer in the kit for nucleic acid library construction of the second aspect of the present disclosure.

According to a seventh aspect of the present disclosure, provided is a nucleic acid library obtained through the method for nucleic acid library construction of the sixth aspect of the present disclosure.

According to an eighth aspect of the present disclosure, provided is a sequencing method, which includes the steps of the method for nucleic acid library construction of the sixth aspect of the present disclosure.

Preferably, the sequencing method includes the following steps: obtaining a nucleic acid library; and sequencing; wherein, the nucleic acid library is obtained by the method for nucleic acid library construction of the sixth aspect of the present disclosure.

Preferably, the sequencing method may further include a step of library quality inspection before the sequencing.

According to a ninth aspect of the present disclosure, provided is use of the adapter composition of the first aspect of the present disclosure, the kit of the second aspect, the sequencing reagent kit of the third aspect, and/or the sequencing system of the fourth aspect.

The adapter composition of the first aspect of the present disclosure and/or the kit of the second aspect of the present disclosure for use in any one of c1) to c6):
c1) Preparation of a nucleic acid library;
c2) Preparation of a product for nucleic acid library construction;
c3) Sequencing;
c4) Preparation of a product for sequencing;
c5) DNA methylation detection; and
c6) Preparation of a product for DNA methylation detection.

Preferably, the nucleic acid library may include DNA methylation library.

The sequencing reagent kit of the third aspect of the present disclosure and/or the sequencing system of the fourth aspect of the present disclosure for use in any one of c3) to c6);
c3) Sequencing;
c4) Preparation of a product for sequencing;
c5) DNA methylation detection; and
c6) Preparation of a product for DNA methylation detection.

The present disclosure is further described in detail below in conjunction with specific examples.

It should be understood that these examples are only used for illustrating the present disclosure and not intended to limit the scope of the present disclosure.

The experimental methods in the following examples are generally conducted under conventional conditions or under conditions recommended by the manufacturer, unless otherwise specified. The materials and reagents used in the examples are commercially available unless otherwise specified.

### Example 1 - Construction and sequencing of human peripheral blood cell-free DNA methylation library

### I. Experimental materials

Cell-free DNA extracted from human peripheral blood plasma (sample name: cfDNA-1).

### II. Experimental steps

1. To a 200-µL PCR tube, 5 ng of cell-free DNA (cfDNA) from human peripheral blood plasma was added, with a total volume of 20 µL.
2. The cfDNA was subjected to Bisulfite treatment and purification using EZ DNA Methylation-Gold Kit (Zymo Research, Cat. No. D5005/D5006).
3. 900 µL of NF water, 300 µL of M-Dilution Buffer, and 50 µL of M-Dissolving Buffer were successively pipetted into a tube containing CT Conversion Reagent powder (which should be briefly centrifuged before opening the cap). The mixture was vortexed frequently at room temperature for 10 min to complete the preparation of the CT Conversion Reagent. The CT Conversion Reagent should be prevented from light exposure and preferably prepared freshly before use. The CT Conversion Reagent could be stored for no more than 1 day at room temperature, no more than 1 week at 4 °C, and no more than 1 month at -20 °C. The CT Conversion Reagent that was not freshly prepared should be pre-warmed to 37 °C before use and vortexed frequently at room temperature for 10 min before use.
4. M-Wash Buffer should be added with an appropriate volume of absolute ethanol as indicated on the bottle label before the M-Wash Buffer was used for the first time, and the mixture should be thoroughly mixed before use. The components shown in Table 1 were added into a new 200-µL PCR tube at room temperature.

**Table 1**

| Components | Volume |
|---|---|
| CT Conversion Reagent | 130 µL |
| cfDNA | 20 µL |
| Total | 150 µL |

5. The 200-µL PCR tube was placed on the PCR apparatus, and the reaction was carried out according to the Bisulfite treatment condition shown in Table 2.

**Table 2**

| Temperature | Time |
|---|---|
| Heated lid | On |
| 98 °C | 10 min |
| 64 °C | 2.5 h |
| 4 °C | Hold |

6. The reaction product obtained in the Step 5 was transferred into a new 1.5-mL centrifuge tube, and 600 µL of M-Binding Buffer was added. The mixture was vortexed and shaken 6 times, 3 seconds each time, and then briefly centrifuged to collect the reaction solution at the bottom of the tube.
7. A Zymo-Spin IC Column was put into a 2-mL Collection Tube. The mixture obtained in the Step 6 was transferred onto the Zymo-Spin IC Column, and then centrifuged at 13,000 rpm for 30 seconds. The waste liquid was discarded, and the Zymo-Spin IC Column was put back into the Collection Tube.
8. 100 µL of M-Wash Buffer was added to the Zymo-Spin IC Column, and then centrifuged at 13,000 rpm for 30 seconds.
9. 200 µL of M-Desulphonation Buffer was added to the Zymo-Spin IC Column. After quickly closing the tube cap, the mixture was incubated at room temperature for 15-20 minutes, and then centrifuged at 13,000 rpm for 30 seconds. The waste liquid was discarded, and then the Zymo-Spin IC Column was put back into the Collection Tube.
10. 200 µL of M-Wash Buffer was added to the Zymo-Spin IC Column, and then centrifuged at 13,000 rpm for 30 seconds. The waste liquid was discarded, and the Zymo-Spin IC Column was put back into the Collection Tube.
11. 200 µL of M-Wash Buffer was added to the Zymo-Spin IC Column, and then centrifuged at 13,000 rpm for 30 seconds. The waste liquid was discarded, and the Zymo-Spin IC Column was put back into the Collection Tube, and then empty-centrifuged at 13,000 rpm for 30 seconds. The Collection Tube was discarded, and the liquid on the outer wall of the Zymo-Spin IC Column was pipetted as much as possible using a pipette, and transferred to a new 1.5-mL centrifuge tube.
12. The cap of the Zymo-Spin IC Column was opened to dry at room temperature for 2 minutes. Then the Zymo-Spin IC Column was placed in another new 1.5-mL centrifuge tube.
13. 8 µL of M-Elution Buffer was slowly added to the center of the filter membrane of the Zymo-Spin IC Column, allowed to stand for 1 minute, then centrifuged at 13,000 rpm for 30 seconds, and the purified product after the Bisulfite treatment was collected in the 1.5-mL centrifuge tube.
14. 1 µL of rSAP (YEASEN, 10322ES72) and 1 µL of 10x rSAP buffer (YEASEN, 10322ES72) were added to the PCR tube, vortexed to mix evenly, and then briefly centrifuged.
15. The PCR tube was placed in the PCR apparatus, incubated at 25°C for 30 minutes, at 65°C for 5 minutes, and at 95°C for 3 minutes. After the reaction was completed, the tube was placed on ice immediately and allowed to stand for 2 minutes.
16. 5 µL of the adapter Mix (10 µM) was added to the PCR tube:
NH₂₋5Ad-uni (5' NH₂ C6-GAACGACATGGCTACGATCCGACTT (SEQ ID NO. 1) - 3'P);
3Ad-Top-NH₂ (5' P-AAGTCGGAGGCCAAGCGG (SEQ ID NO. 2) -3' NH₂ C6).

17. The ligation reaction mixture was prepared according to Table 3.

**Table 3**

| Components | Volume for a single reaction |
|---|---|
| 1X RtcB Reaction Buffer (NEB, M0458S) | 5 µL |
| ET SSB (NEB, M2401S) | 0.5 µL |
| 1 mM GTP (NEB, M0458S) | 5 µL |
| 10 mM MnCl₂ (NEB, M0458S) | 5 µL |
| 100 mM ATP | 0.5 µL |
| 50% PEG8000(NEB, M0204S) | 15 µL |
| RtcB ligase (NEB, M0458S) | 2 µL |
| T4 RNA ligase1(NEB, M0204S) | 2 µL |
| Total | 35 µL |

18. 35 µL of the prepared ligation reaction solution was added to the PCR tube, then vortexed to mix evenly and centrifuged briefly.
19. The PCR tube was put into the PCR apparatus, and the program was set as follows: incubated at 37 °C for 30 minutes, and held at 4 °C.
20. After the reaction was completed, the liquid in the PCR tube was collected to the bottom of the tube by brief centrifugation.
21. 50 µL of the purification magnetic beads was aspirated into a 1.5-mL centrifuge tube. The reaction product was transferred into the centrifuge tube, and gently blown using a pipette for at least 10 times until all the magnetic beads were suspended. For the last blowing, ensure that all the liquid and magnetic beads in the pipette tip were dispensed into the centrifuge tube.
22. After being incubated at room temperature for 5 minutes and briefly centrifuged, the centrifuge tube was placed on the magnetic rack and allowed to stand for 2 to 5 minutes until the liquid became clear. The supernatant was carefully aspirated with a pipette and discarded.
23. The centrifuge tube was held on the magnetic rack; and 200 µL of freshly prepared 80% ethanol was added to rinse the magnetic beads and the tube wall. After allowing to stand for 30 seconds, the supernatant was carefully aspirated and discarded.
24. The previous step was repeated, and the liquid in the tube was aspirated as much as possible. When there was a small amount of residue on the tube wall, the centrifuge tube could be centrifuged briefly. After removing from the magnetic rack, the liquid at the bottom of the tube was aspirated using a pipette with a small volume range.
25. The centrifuge tube was held on the magnetic rack; and the cap of the centrifuge tube was opened and dried at room temperature until there was no reflection on the surface of the magnetic beads.
26. The centrifuge tube was removed from the magnetic rack, and 20 µL of TE Buffer (AMBION, AM9858) was added for elution. The mixture was gently blown using a pipette for at least 10 times until mixed thoroughly.
27. The mixture was incubated at room temperature for 5 minutes.
28. The centrifuge tube was placed on the magnetic rack after brief centrifugation, and allowed to stand for 2 to 5 minutes until the liquid became clear. 19 µL of the supernatant was aspirated using a pipette to a new 1.5-mL centrifuge tube.
29. The PCR reaction solution was prepared on ice according to Table 4.

**Table 4**

| Component | Volume for a single reaction |
|---|---|
| KAPA Uracil + HiFi HotStart ReadyMix (KAPA Biosystems, KK2801) | 25 µL |
| PCR Barcode Primer Mix | 6 µL |
| Total | 31 µL |

PCR Barcode Primer Mix (20µM):
PCR primer1 (10 µM): 5' P-GAACGACATGGCTACGA-3' (SEQ ID NO. 3);
PCR Barcode primer (10 µM): 5'-TGTGAGCCAAGGAGTTGATCGGACCTATTGTCTTCCT AAGACCGCTTGGCCTCCGACTT-3' (SEQ ID NO. 4); with the underlined part representing a sample tag.

30. 31 µL of the prepared PCR reaction solution was aspirated with a pipette and added to the PCR tube containing the purified product. The tube was vortexed and shaken for 3 times, 3 seconds each time, and then briefly centrifuged to collect the reaction solution at the bottom of the tube.
31. The PCR tube was placed on the PCR apparatus, and the PCR reaction was carried out according to the condition in Table 5.

**Table 5**

| Temperature | Time | Cycles |
|---|---|---|
| 105 °C for heated lid | on | |
| 95 °C | 3 min | 1 |
| 98 °C | 20 s | 10 |
| 60 °C | 30 s | |
| 72 °C | 30 s | |
| 72 °C | 10 min | 1 |
| 4 °C | Hold | |

32. After the reaction was completed, the tube was briefly centrifuged to collect the reaction solution at the bottom of the tube.
33. 50 µL of the purification magnetic beads were aspirated into a 1.5-mL centrifuge tube. The reaction product was transferred into the centrifuge tube, and the magnetic beads were gently blown using a pipette for at least 10 times until all the magnetic beads were suspended. For the last blowing, ensure that all the liquid and magnetic beads in the pipette tip were dispensed into the centrifuge tube.
34. After being incubated at room temperature for 5 minutes and briefly centrifuged, the centrifuge tube was placed on the magnetic rack and allowed to stand for 2 to 5 minutes until the liquid became clear. The supernatant was carefully aspirated with a pipette and discarded.
35. The centrifuge tube was held on the magnetic rack; and 200 µL of freshly prepared 80% ethanol was added to rinse the magnetic beads and the tube wall. After allowing to stand for 30 seconds, the supernatant was carefully aspirated and discarded.
36. The previous step was repeated, and the liquid in the tube was aspirated as much as possible. When there was a small amount of residue on the tube wall, the centrifuge tube could be centrifuged briefly. After removing from the magnetic rack, the liquid at the bottom of the tube was aspirated using a pipette with a small volume range.
37. The centrifuge tube was held on the magnetic rack; and the cap of the centrifuge tube was opened and dried at room temperature until there was no reflection on the surface of the magnetic beads.
38. The centrifuge tube was removed from the magnetic rack, and 32 µL of TE Buffer (AMBION, AM9858) was added for elution. The mixture was gently blown using a pipette for at least 10 times until mixed thoroughly.
39. The mixture was incubated at room temperature for 5 minutes.
40. The centrifuge tube was placed on the magnetic rack after brief centrifugation, and allowed to stand for 2 to 5 minutes until the liquid became clear. 30 µL of the supernatant was aspirated using a pipette to a new 1.5-mL centrifuge tube.
41. The purified PCR product library was quantified using Qubit^{®} dsDNA HS Assay Kit (Theromo Fisher, Q32854) fluorescence quantitative kit according to the instruction of the Assay Kit.
42. 280 ng of the PCR product was transferred to a new 0.2-mL PCR tube, and TE Buffer (AMBION, AM9858) was added to make up the total volume to 48 µL.
43. The PCR tube was placed on the PCR apparatus, and the reaction was carried out according to the condition in Table 6.

**Table 6**

| Temperature | Time |
|---|---|
| 105 °C for heated lid | On |
| 95 °C | 3 min |

44. After the reaction was completed, the PCR tube was immediately placed on ice and allowed to stand for 2 minutes. Then, the single-stranded cyclization reaction solution was added.
45. MGI cyclization reagent kit (MGI, 1000005260) was used to prepare the single-stranded cyclization reaction solution on ice in advance according to the formula shown in Table 7 below.

**Table 7**

| Components | Volume for a single reaction |
|---|---|
| Splint Buffer | 11.6 µL |
| DNA Rapid Ligase | 0.5 µL |
| Total | µL |

46. 12.1 µL of the prepared single-stranded cyclization reaction solution was aspirated with a pipette and added to the PCR tube. The tube was vortexed and shaken for 3 times, 3 seconds each time, and then briefly centrifuged to collect the reaction solution at the bottom of the tube.
47. The PCR tube was placed on the PCR apparatus, and the reaction was carried out according to the condition in Table 8.

**Table 8**

| Temperature | Time |
|---|---|
| 45 °C for heated lid | On |
| 37 °C | 30 min |
| 4 °C | Hold |

48. After the reaction was completed, the PCR tube was briefly centrifuged and placed on ice, and the next step of reaction was carried out immediately.
49. MGI cyclization reagent kit (MGI, 1000005260) was used to prepare the enzymatic digestion reaction solution on ice in advance according to the formula shown in Table 9 below.

**Table 9**

| Components | Volume for a single reaction |
|---|---|
| Digestion Buffer | 1.4 µL |
| Digestion Enzyme | 2.6 µL |
| Total | 4 µL |

50. 4 µL of the prepared enzymatic digestion reaction solution was aspirated with a pipette and added to the PCR tube. The tube was vortexed and shaken for 3 times, 3 seconds each time, and then briefly centrifuged to collect the reaction solution at the bottom of the tube.
51. The PCR tube was placed on the PCR apparatus, and the reaction was carried out according to the condition in Table 10.

**Table 10**

| Temperature | Time |
|---|---|
| 45 °C for heated lid | On |
| 37 °C | 30 min |
| 4 °C | Hold |

52. After the reaction was completed, the tube was briefly centrifuged to collect the reaction solution at the bottom of the tube.
53. 170 µL of the purification magnetic beads were aspirated into a 1.5-mL centrifuge tube. The reaction product was transferred into the centrifuge tube, and the magnetic beads were gently blown using a pipette for at least 10 times until all the magnetic beads were suspended. For the last blowing, ensure that all the liquid and magnetic beads in the pipette tip were dispensed into the centrifuge tube.
54. After being incubated at room temperature for 10 minutes and briefly centrifuged, the centrifuge tube was placed on the magnetic rack and allowed to stand for 2 to 5 minutes until the liquid became clear. The supernatant was carefully aspirated with a pipette and discarded.
55. The centrifuge tube was held on the magnetic rack; and 300 µL of freshly prepared 80% ethanol was added to rinse the magnetic beads and the tube wall. After allowing to stand for 30 seconds, the supernatant was carefully aspirated and discarded.
56. The previous step was repeated, and the liquid in the tube was aspirated as much as possible. When there was a small amount of residue on the tube wall, the centrifuge tube could be centrifuged briefly. After removing from the magnetic rack, the liquid at the bottom of the tube was aspirated using a pipette with a small volume range.
57. The centrifuge tube was held on the magnetic rack; and the cap of the centrifuge tube was opened and dried at room temperature until there was no reflection on the surface of the magnetic beads.
58. The centrifuge tube was removed from the magnetic rack, and 22 µL of TE Buffer (AMBION, AM9858) was added for elution. The mixture was gently blown using a pipette for at least 10 times until mixed thoroughly.
59. The mixture was incubated at room temperature for 10 minutes.
60. The centrifuge tube was placed on the magnetic rack after brief centrifugation, and allowed to stand for 2 to 5 minutes until the liquid became clear. 20 µL of the supernatant was aspirated using a pipette to a new 1.5-mL centrifuge tube.
61. The digested and purified PCR product was quantified using Qubit^{®} ssDNA Assay Kit (Thermo Fisher, Q10212) fluorescence quantitative kit according to the instruction of the Assay Kit.
62. The sequencing was performed on the MGISEQ-2000 sequencer using MGISEQ-2000 high-throughput sequencing kit (PE100) (MGI, 1000012536), and DNB preparation and sequencing were carried out according to the instructions.

### III. Experimental results

Using 5 ng of cfDNA as the sample, after sulfite treatment, the library construction was performed according the present method. Under 10 cycles of PCR, a PCR product library of 1458.3 ng was obtained, which indicated a high library construction yield (see Table 11).

**Table 11**

| Sample name | Sample type | DNA input amount | PCR product library yield | Single-strand cyclization library yield |
|---|---|---|---|---|
| cfDNA-1 | cfDNA | 5 ng | 1458.3 ng | 36.4 ng |

The obtained PCR product library was subjected to single-strand cyclization to obtain the corresponding single-strand cyclization library (36.4 ng). The single-strand cyclization library was sequenced on the DNBSEQ sequencing platform, with the sample being loaded into one lane of the MGISEQ-2000 sequencing chip. The results are shown in Table 12 and Figure 3. The obtained final sequencing data could reach 430 M, 90.53% of which reached the sequencing quality value Q30. The base distribution graph (Figure 3) also showed that the balanced base distribution of the library, indicating that there was basically no contamination from the adapters or other fixing sequences. The standard analysis results showed low repetition rate (7.0%), indicating that the high utilization efficiency of the nucleic acid template even under low sample input.

**Table 12**

| | |
|---|---|
| Sample name | cfDNA-1 |
| Raw sequenced reads | 860725398 |
| Proportion of sequenced reads reaching Q30 | 90.53% |
| Filtered sequenced reads | 786703013.8 |
| Proportion of filtered sequenced reads | 91.4% |
| Coverage | 97.8% |
| Average depth | 21.36 |
| Coverage at depth of 20x or higher | 54.90% |
| Coverage at depth of 10x or higher | 86.11% |
| Coverage at depth of 40x or higher | 95.49% |
| Insert fragment size | 148 |
| Duplication rate | 7.0% |
| Mapped rate | 87.60% |
| Unique mapped rate | 84.50% |

### Example 2 - Construction of human peripheral blood cell-free DNA methylation library

### I. Experimental materials

Cell-free DNA extracted from human peripheral blood plasma (sample name: cfDNA-2).

### II. Experimental steps

1. To a 200-µL PCR tube, 5 ng of cell-free DNA (cfDNA-2) from human peripheral blood plasma was added, with a total volume of 20 µL.
2. The cfDNA was subjected to Bisulfite treatment and purification using EZ DNA Methylation-Gold Kit (Zymo Research, Cat. No. D5005/D5006).
3. 900 µL of NF water, 300 µL of M-Dilution Buffer, and 50 µL of M-Dissolving Buffer were successively pipetted into a tube containing CT Conversion Reagent powder (which should be briefly centrifuged before opening the cap). The mixture was vortexed frequently at room temperature for 10 min to complete the preparation of the CT Conversion Reagent. The CT Conversion Reagent should be prevented from light exposure and preferably prepared freshly before use. The CT Conversion Reagent could be stored for no more than 1 day at room temperature, no more than 1 week at 4 °C, and no more than 1 month at -20 °C. The CT Conversion Reagent that was not freshly prepared should be pre-warmed to 37 °C before use and vortexed frequently at room temperature for 10 min before use.
4. M-Wash Buffer should be added with an appropriate volume of absolute ethanol as indicated on the bottle label before the M-Wash Buffer was used for the first time, and the mixture should be thoroughly mixed before use. The components shown in Table 1 were added into a new 200-µL PCR tube at room temperature.
5. The 200-µL PCR tube was placed on the PCR apparatus, and the reaction was carried out according to the Bisulfite treatment condition shown in Table 2.
6. The reaction product obtained in the Step 5 was transferred into a new 1.5-mL centrifuge tube, and 600 µL of M-Binding Buffer was added. The mixture was vortexed and shaken 6 times, 3 seconds each time, and then briefly centrifuged to collect the reaction solution at the bottom of the tube.
7. A Zymo-Spin IC Column was put into a 2-mL Collection Tube. The mixture obtained in the Step 6 was transferred onto the Zymo-Spin IC Column, and then centrifuged at 13,000 rpm for 30 seconds. The waste liquid was discarded, and the Zymo-Spin IC Column was put back into the Collection Tube.
8. 100 µL of M-Wash Buffer was added to the Zymo-Spin IC Column, and then centrifuged at 13,000 rpm for 30 seconds.
9. 200 µL of M-Desulphonation Buffer was added to the Zymo-Spin IC Column. After quickly closing the tube cap, the mixture was incubated at room temperature for 15-20 minutes, and then centrifuged at 13,000 rpm for 30 seconds. The waste liquid was discarded, and then the Zymo-Spin IC Column was put back into the Collection Tube.
10. 200 µL of M-Wash Buffer was added to the Zymo-Spin IC Column, and then centrifuged at 13,000 rpm for 30 seconds. The waste liquid was discarded, and the Zymo-Spin IC Column was put back into the Collection Tube.
11. 200 µL of M-Wash Buffer was added to the Zymo-Spin IC Column, and then centrifuged at 13,000 rpm for 30 seconds. The waste liquid was discarded, and the Zymo-Spin IC Column was put back into the Collection Tube, and then empty-centrifuged at 13,000 rpm for 30 seconds. The Collection Tube was discarded, and the liquid on the outer wall of the Zymo-Spin IC Column was pipetted as much as possible using a pipette, and transferred to a new 1.5-mL centrifuge tube.
12. The cap of the Zymo-Spin IC Column was opened to dry at room temperature for 2 minutes. Then the Zymo-Spin IC Column was placed in another new 1.5-mL centrifuge tube.
13. 8 µL of M-Elution Buffer was slowly added to the center of the filter membrane of the Zymo-Spin IC Column, allowed to stand for 1 minute, then centrifuged at 13,000 rpm for 30 seconds, and the purified product after the Bisulfite treatment was collected in the 1.5-mL centrifuge tube.
14. 1 µL of rSAP (YEASEN, 10322ES72) and 1 µL of 10x rSAP buffer (YEASEN, 10322ES72) were added to the PCR tube, vortexed to mix evenly, and then briefly centrifuged.
15. The PCR tube was placed in the PCR apparatus, incubated at 25°C for 30 minutes, at 65°C for 5 minutes, and at 95°C for 3 minutes. After the reaction was completed, the tube was placed on ice immediately and allowed to stand for 2 minutes.
16. 1 µL of the adapter Mix (50 µM) was added to the PCR tube:
NH₂₋5Ad-uni (5' NH₂ C6-GAACGACATGGCTACGATCCGACTT (SEQ ID NO. 1) -3' P);
3Ad-Top-NH₂ (5' P-AAGTCGGAGGCCAAGCGG (SEQ ID NO. 2) -3' NH₂ C6).

17. The ligation reaction mixture was prepared according to Table 13.

**Table 13**

| Components | Volume for a single reaction |
|---|---|
| 10X RtcB Reaction Buffer (NEB, M0458S) | 3 µL |
| ET SSB (NEB, M2401S) | 0.5 µL |
| 1 mM GTP (NEB, M0458S) | 3 µL |
| 10 mM MnCl₂ (NEB, M0458S) | 1.5 µL |
| 100 mM ATP | 0.5 µL |
| 50% PEG8000 (NEB, M0204S) | 9 µL |
| RtcB ligase (NEB, M0458S) | 1 µL |
| T4 RNA ligase1 (NEB, M0204S) | 0.5 µL |
| Total | 19 µL |

18. 19 µL of the prepared ligation reaction solution was added to the PCR tube, then vortexed to mix evenly and centrifuged briefly.
19. The PCR tube was put into the PCR apparatus, and the program was set as follows: incubated at 16 °C for 60 minutes, and held at 4 °C.
20. After the reaction was completed, the liquid in the PCR tube was collected to the bottom of the tube by brief centrifugation.
21. 30 µL of the purification magnetic beads was aspirated into a 1.5-mL centrifuge tube. The reaction product was transferred into the centrifuge tube, and gently blown using a pipette for at least 10 times until all the magnetic beads were suspended. For the last blowing, ensure that all the liquid and magnetic beads in the pipette tip were dispensed into the centrifuge tube.
22. After being incubated at room temperature for 5 minutes and briefly centrifuged, the centrifuge tube was placed on the magnetic rack and allowed to stand for 2 to 5 minutes until the liquid became clear. The supernatant was carefully aspirated with a pipette and discarded.
23. The centrifuge tube was held on the magnetic rack; and 200 µL of freshly prepared 80% ethanol was added to rinse the magnetic beads and the tube wall. After allowing to stand for 30 seconds, the supernatant was carefully aspirated and discarded.
24. The previous step was repeated, and the liquid in the tube was aspirated as much as possible. When there was a small amount of residue on the tube wall, the centrifuge tube could be centrifuged briefly. After removing from the magnetic rack, the liquid at the bottom of the tube was aspirated using a pipette with a small volume range.
25. The centrifuge tube was held on the magnetic rack; and the cap of the centrifuge tube was opened and dried at room temperature until there was no reflection on the surface of the magnetic beads.
26. The centrifuge tube was removed from the magnetic rack, and 20 µL of TE Buffer (AMBION, AM9858) was added for elution. The mixture was gently blown using a pipette for at least 10 times until mixed thoroughly.
27. The mixture was incubated at room temperature for 5 minutes.
28. The centrifuge tube was placed on the magnetic rack after brief centrifugation, and allowed to stand for 2 to 5 minutes until the liquid became clear. 19 µL of the supernatant was aspirated using a pipette to a new 1.5-mL centrifuge tube.
29. The PCR reaction solution was prepared on ice according to Table 4; and the PCR Barcode Primer Mix was the same as that in Example 1.
30. 31 µL of the prepared PCR reaction solution was aspirated with a pipette and added to the PCR tube containing the purified product. The tube was vortexed and shaken for 3 times, 3 seconds each time, and then briefly centrifuged to collect the reaction solution at the bottom of the tube.
31. The PCR tube was placed on the PCR apparatus, and the PCR reaction was carried out according to the condition in Table 5.
32. After the reaction was completed, the tube was briefly centrifuged to collect the reaction solution at the bottom of the tube.
33. 50 µL of the purification magnetic beads were aspirated into a 1.5-mL centrifuge tube. The reaction product was transferred into the centrifuge tube, and the magnetic beads were gently blown using a pipette for at least 10 times until all the magnetic beads were suspended. For the last blowing, ensure that all the liquid and magnetic beads in the pipette tip were dispensed into the centrifuge tube.
34. After being incubated at room temperature for 5 minutes and briefly centrifuged, the centrifuge tube was placed on the magnetic rack and allowed to stand for 2 to 5 minutes until the liquid became clear. The supernatant was carefully aspirated with a pipette and discarded.
35. The centrifuge tube was held on the magnetic rack; and 200 µL of freshly prepared 80% ethanol was added to rinse the magnetic beads and the tube wall. After allowing to stand for 30 seconds, the supernatant was carefully aspirated and discarded.
36. The previous step was repeated, and the liquid in the tube was aspirated as much as possible. When there was a small amount of residue on the tube wall, the centrifuge tube could be centrifuged briefly. After removing from the magnetic rack, the liquid at the bottom of the tube was aspirated using a pipette with a small volume range.
37. The centrifuge tube was held on the magnetic rack; and the cap of the centrifuge tube was opened and dried at room temperature until there was no reflection on the surface of the magnetic beads.
38. The centrifuge tube was removed from the magnetic rack, and 32 µL of TE Buffer (AMBION, AM9858) was added for elution. The mixture was gently blown using a pipette for at least 10 times until mixed thoroughly.
39. The mixture was incubated at room temperature for 5 minutes.
40. The centrifuge tube was placed on the magnetic rack after brief centrifugation, and allowed to stand for 2 to 5 minutes until the liquid became clear. 30 µL of the supernatant was aspirated using a pipette to a new 1.5-mL centrifuge tube.
41. The purified PCR product library was quantified using Qubit^{®} dsDNA HS Assay Kit (Theromo Fisher, Q32854) fluorescence quantitative kit according to the instruction of the Assay Kit. The concentration of the obtained PCR library from the cfDNA-2 sample was 39.5 ng/µL. Therefore, the library yield of the present library construction method was 1,185 ng, indicating that the high library yield as well as the high utilization rate of cfDNA template.

### Example 3 - Construction of human peripheral blood cell-free DNA methylation library

### I. Experimental materials

Cell-free DNA extracted from human peripheral blood plasma (sample name: cfDNA-3).

### II. Experimental steps

1. To a 200-µL PCR tube, 5 ng of cell-free DNA (cfDNA-3) from human peripheral blood plasma was added, with a total volume of 20 µL.
2. The cfDNA was subjected to Bisulfite treatment and purification using EZ DNA Methylation-Gold Kit (Zymo Research, Cat. No. D5005/D5006).
3. 900 µL of NF water, 300 µL of M-Dilution Buffer, and 50 µL of M-Dissolving Buffer were successively pipetted into a tube containing CT Conversion Reagent powder (which should be briefly centrifuged before opening the cap). The mixture was vortexed frequently at room temperature for 10 min to complete the preparation of the CT Conversion Reagent. The CT Conversion Reagent should be prevented from light exposure and preferably prepared freshly before use. The CT Conversion Reagent could be stored for no more than 1 day at room temperature, no more than 1 week at 4 °C, and no more than 1 month at -20 °C. The CT Conversion Reagent that was not freshly prepared should be pre-warmed to 37 °C before use and vortexed frequently at room temperature for 10 min before use.
4. M-Wash Buffer should be added with an appropriate volume of absolute ethanol as indicated on the bottle label before the M-Wash Buffer was used for the first time, and the mixture should be thoroughly mixed before use. The components shown in Table 1 were added into a new 200-µL PCR tube at room temperature.
5. The 200-µL PCR tube was placed on the PCR apparatus, and the reaction was carried out according to the Bisulfite treatment condition shown in Table 2.
6. The reaction product obtained in the Step 5 was transferred into a new 1.5-mL centrifuge tube, and 600 µL of M-Binding Buffer was added. The mixture was vortexed and shaken 6 times, 3 seconds each time, and then briefly centrifuged to collect the reaction solution at the bottom of the tube.
7. A Zymo-Spin IC Column was put into a 2-mL Collection Tube. The mixture obtained in the Step 6 was transferred onto the Zymo-Spin IC Column, and then centrifuged at 13,000 rpm for 30 seconds. The waste liquid was discarded, and the Zymo-Spin IC Column was put back into the Collection Tube.
8. 100 µL of M-Wash Buffer was added to the Zymo-Spin IC Column, and then centrifuged at 13,000 rpm for 30 seconds.
9. 200 µL of M-Desulphonation Buffer was added to the Zymo-Spin IC Column. After quickly closing the tube cap, the mixture was incubated at room temperature for 15-20 minutes, and then centrifuged at 13,000 rpm for 30 seconds. The waste liquid was discarded, and then the Zymo-Spin IC Column was put back into the Collection Tube.
10. 200 µL of M-Wash Buffer was added to the Zymo-Spin IC Column, and then centrifuged at 13,000 rpm for 30 seconds. The waste liquid was discarded, and the Zymo-Spin IC Column was put back into the Collection Tube.
11. 200 µL of M-Wash Buffer was added to the Zymo-Spin IC Column, and then centrifuged at 13,000 rpm for 30 seconds. The waste liquid was discarded, and the Zymo-Spin IC Column was put back into the Collection Tube, and then empty-centrifuged at 13,000 rpm for 30 seconds. The Collection Tube was discarded, and the liquid on the outer wall of the Zymo-Spin IC Column was pipetted as much as possible using a pipette, and transferred to a new 1.5-mL centrifuge tube.
12. The cap of the Zymo-Spin IC Column was opened to dry at room temperature for 2 minutes. Then the Zymo-Spin IC Column was placed in another new 1.5-mL centrifuge tube.
13. 8 µL of M-Elution Buffer was slowly added to the center of the filter membrane of the Zymo-Spin IC Column, allowed to stand for 1 minute, then centrifuged at 13,000 rpm for 30 seconds, and the purified product after the Bisulfite treatment was collected in the 1.5-mL centrifuge tube.
14. 1 µL of rSAP (YEASEN, 10322ES72) and 1 µL of 10x rSAP buffer (YEASEN, 10322ES72) were added to the PCR tube, vortexed to mix evenly, and then briefly centrifuged.
15. The PCR tube was placed in the PCR apparatus, incubated at 25°C for 30 minutes, at 65°C for 5 minutes, and at 95°C for 3 minutes. After the reaction was completed, the tube was placed on ice immediately and allowed to stand for 2 minutes.
16. 5 µL of the adapter Mix (10 µM) was added to the PCR tube:
NH₂₋5Ad-uni (5' NH₂ C6-GAACGACATGGCTACGATCCGACTT (SEQ ID NO. 1) -3' P);
3Ad-Top-NH₂ (5' P-AAGTCGGAGGCCAAGCGG (SEQ ID NO. 2) -3' NH₂ C6).

17. The ligation reaction mixture was prepared according to Table 14.

**Table 14**

| Components | Volume for a single reaction |
|---|---|
| 1X RtcB Reaction Buffer (NEB, M0458S) | 5 µL |
| ET SSB (NEB, M2401S) | 0.5 µL |
| 1 mM GTP (NEB, M0458S) | 5 µL |
| 10 mM MnCl₂ (NEB, M0458S) | 5 µL |
| 50% PEG8000 (NEB, M0204S) | 15 µL |
| Pho RtcB ligase | 2 µL |
| CircLigase ssDNA Ligase (Lucigen, CL4111K) | 2.5 µL |
| Total | 35 µL |

| | |
|---|---|
| Note: Pho RtcB ligase was the purified expression product of RtcB gene of the extremely thermophilic archaeon *Pyrococcus horikoshii* (UniProt accession number A0A832T3E6). | |

18. 35 µL of the prepared ligation reaction solution was added to the PCR tube, then vortexed to mix evenly and centrifuged briefly.
19. The PCR tube was put into the PCR apparatus, and the program was set as follows: incubated at 65 °C for 30 minutes, and held at 4 °C.
20. After the reaction was completed, the liquid in the PCR tube was collected to the bottom of the tube by brief centrifugation.
21. 50 µL of the purification magnetic beads was aspirated into a 1.5-mL centrifuge tube. The reaction product was transferred into the centrifuge tube, and gently blown using a pipette for at least 10 times until all the magnetic beads were suspended. For the last blowing, ensure that all the liquid and magnetic beads in pipette tip were dispensed into the centrifuge tube.
22. After being incubated at room temperature for 5 minutes and briefly centrifuged, the centrifuge tube was placed on the magnetic rack and allowed to stand for 2 to 5 minutes until the liquid became clear. The supernatant was carefully aspirated with a pipette and discarded.
23. The centrifuge tube was held on the magnetic rack; and 200 µL of freshly prepared 80% ethanol was added to rinse the magnetic beads and the tube wall. After allowing to stand for 30 seconds, the supernatant was carefully aspirated and discarded.
24. The previous step was repeated, and the liquid in the tube was aspirated as much as possible. When there was a small amount of residue on the tube wall, the centrifuge tube could be centrifuged briefly. After removing from the magnetic rack, the liquid at the bottom of the tube was aspirated using a pipette with a small volume range.
25. The centrifuge tube was held on the magnetic rack; and the cap of the centrifuge tube was opened and dried at room temperature until there was no reflection on the surface of the magnetic beads.
26. The centrifuge tube was removed from the magnetic rack, and 20 µL of TE Buffer (AMBION, AM9858) was added for elution. The mixture was gently blown using a pipette for at least 10 times until mixed thoroughly.
27. The mixture was incubated at room temperature for 5 minutes.
28. The centrifuge tube was placed on the magnetic rack after brief centrifugation, and allowed to stand for 2 to 5 minutes until the liquid became clear. 19 µL of the supernatant was aspirated using a pipette to a new 1.5-mL centrifuge tube.
29. The PCR reaction solution was prepared on ice according to Table 4; and the PCR Barcode Primer Mix was the same as that in Example 1.
30. 31 µL of the prepared PCR reaction solution was aspirated with a pipette and added to the PCR tube containing the purified product. The tube was vortexed and shaken for 3 times, 3 seconds each time, and then briefly centrifuged to collect the reaction solution at the bottom of the tube.
31. The PCR tube was placed on the PCR apparatus, and the PCR reaction was carried out according to the condition in Table 5.
32. After the reaction was completed, the tube was briefly centrifuged to collect the reaction solution at the bottom of the tube.
33. 50 µL of the purification magnetic beads were aspirated into a 1.5-mL centrifuge tube. The reaction product was transferred into the centrifuge tube, and the magnetic beads were gently blown using a pipette for at least 10 times until all the magnetic beads were suspended. For the last blowing, ensure that all the liquid and magnetic beads in the pipette tip were dispensed into the centrifuge tube.
34. After being incubated at room temperature for 5 minutes and briefly centrifuged, the centrifuge tube was placed on the magnetic rack and allowed to stand for 2 to 5 minutes until the liquid became clear. The supernatant was carefully aspirated with a pipette and discarded.
35. The centrifuge tube was held on the magnetic rack; and 200 µL of freshly prepared 80% ethanol was added to rinse the magnetic beads and the tube wall. After allowing to stand for 30 seconds, the supernatant was carefully aspirated and discarded.
36. The previous step was repeated, and the liquid in the tube was aspirated as much as possible. When there was a small amount of residue on the tube wall, the centrifuge tube could be centrifuged briefly. After removing from the magnetic rack, the liquid at the bottom of the tube was aspirated using a pipette with a small volume range.
37. The centrifuge tube was held on the magnetic rack; and the cap of the centrifuge tube was opened and dried at room temperature until there was no reflection on the surface of the magnetic beads.
38. The centrifuge tube was removed from the magnetic rack, and 32 µL of TE Buffer (AMBION, AM9858) was added for elution. The mixture was gently blown using a pipette for at least 10 times until mixed thoroughly.
39. The mixture was incubated at room temperature for 5 minutes.
40. The centrifuge tube was placed on the magnetic rack after brief centrifugation, and allowed to stand for 2 to 5 minutes until the liquid became clear. 30 µL of the supernatant was aspirated using a pipette to a new 1.5-mL centrifuge tube.
41. The purified PCR product library was quantified using Qubit^{®} dsDNA HS Assay Kit (Theromo Fisher, Q32854) fluorescence quantitative kit according to the instruction of the Assay Kit. The concentration of the obtained PCR library from the cfDNA-2 sample was 43 ng/ µL. Therefore, the library yield of the present library construction method was 1,290 ng, indicating that the high library yield as well as the high utilization rate of cfDNA template.

The foregoing examples are preferred embodiments of the present disclosure, but the embodiments of the present disclosure are not limited to the above examples. Any changes, modifications, substitutions, combinations, simplifications made without departing from the technical solutions of the present disclosure should be considered as equivalent substitutions and should fall within the protection scope of the present disclosure.

## Claims

1. An adapter composition, comprising:
a 5' end adapter comprising a first oligonucleotide, wherein the first oligonucleotide of the 5' end adapter has a first modifying group at the 3' end, the first oligonucleotide of the 5' end adapter has a first blocking group at the 5' end, and the first modifying group comprises a phosphate group;
a first ligase, wherein the first ligase is capable of ligating the first modifying group at the 3' end of the first oligonucleotide of the 5' end adapter to a 5'-hydroxyl end of a template nucleic acid;
a 3' end adapter comprising a second oligonucleotide, wherein the second oligonucleotide of the 3' end adapter has a second blocking group at the 3' end, the second oligonucleotide of the 3' end adapter has a second modifying group at the 5' end, and the second modifying group comprises a phosphate group; and
a second ligase, wherein the second ligase is capable of ligating the second modifying group at the 5' end of the second oligonucleotide of the 3' end adapter to a 3'-hydroxyl end of the template nucleic acid.

2. The adapter composition according to claim 1, wherein:
the first modifying group is at least one modification selected from the group consisting of phosphorylation modification, 2'3' cyclic phosphate modification, and pre-guanosine modification.

3. The adapter composition according to claim 1, wherein:
the second modifying group is at least one modification selected from the group consisting of phosphorylation modification, and adenylation modification.

4. The adapter composition according to claim 1, wherein:
the 5' end adapter further comprises a third oligonucleotide, the third oligonucleotide is in complementary with the first oligonucleotide to form a double strand, and a length of the third oligonucleotide is greater than, equal to or less than a length of the first oligonucleotide;
preferably, the difference between the base numbers of the third oligonucleotide and the first oligonucleotide is greater than or equal to 2; and
preferably, the third oligonucleotide has a third blocking group at the 5' end, and the third oligonucleotide has a fourth blocking group at the 3' end.

5. The adapter composition according to claim 1 or 4, wherein:
the 3' end adapter further comprises a fourth oligonucleotide, the fourth oligonucleotide is in complementary with the second oligonucleotide to form a double strand;
preferably, a length of the fourth oligonucleotide is greater than, equal to or less than a length of the second oligonucleotide; and
preferably, the fourth oligonucleotide has a fifth blocking group at the 5' end, and the fourth oligonucleotide has a sixth blocking group at the 3' end.

6. The adapter composition according to claim 1, wherein:
the adapter composition further comprises: a phosphatase.

7. The adapter composition according to claim 1, wherein:
the first ligase is at least one selected from the group consisting of a RtcB ligase, an active fragment of RtcB ligase, a derivative of RtcB ligase, and an analog of RtcB ligase.

8. The adapter composition according to claim 3, wherein:
when the 5' end of the second oligonucleotide of the 3' end adapter has a phosphorylation modification, the second ligase comprises at least one of T4 RNA ligase 1, T4 DNA ligase, TS2126 RNA ligase, T3 DNA ligase, *Escherichia coli* DNA ligase, single-stranded DNA/RNA circligase, T4 RNA ligase 2, and Taq DNA ligase; or
when the 5' end of the second oligonucleotide of the 3' end adapter has an adenylation modification, the second ligase comprises at least one of T4 RNA ligase 1, T4 RNA ligase 2, T4 RNA ligase 2 (truncated K227Q), T4 RNA ligase 2 (truncated KQ), thermostable 5' App DNA/RNA ligase, TS2126 RNA ligase, T3 DNA ligase, *Escherichia coli* DNA ligase, single-stranded DNA/RNA circligase, and Taq DNA ligase.

9. The adapter composition according to claim 1, 4 or 5, wherein:
the first blocking group is a modification selected from the group consisting of amino modification, dideoxy modification, spacer modification, and phosphate modification;
preferably, the second blocking group is a modification selected from the group consisting of amino modification, dideoxy modification, spacer modification, and phosphate modification;
preferably, the third blocking group is a modification selected from the group consisting of amino modification, dideoxy modification, spacer modification, and phosphate modification;
preferably, the fourth blocking group is a modification selected from the group consisting of amino modification, dideoxy modification, spacer modification, and phosphate modification;
preferably, the fifth blocking group is a modification selected from the group consisting of amino modification, dideoxy modification, spacer modification, and phosphate modification; and
preferably, the sixth blocking group is a modification selected from the group consisting of amino modification, dideoxy modification, spacer modification, and phosphate modification.

10. The adapter composition according to claim 1, wherein:
the adapter composition further comprises: a first cofactor, and first cofactor comprises triphosphate and manganese ion;
preferably, the adapter composition further comprises: a second cofactor, and the second cofactor comprises at least one of ATP and NAD, and a metal ion;
preferably, the adapter composition further comprises: a substance for nucleic acids fragment;
preferably, the adapter composition further comprises: a substance for nucleic acid denaturation;
preferably, when the adapter composition is used for constructing a DNA methylation library, the adapter composition further comprises: a DNA methylation modification conversion reagent; and
preferably, the adapter composition further comprises: a single-strand binding protein.

11. The adapter composition according to claim 1, wherein:
the first oligonucleotide of the 5' end adapter and/or the second oligonucleotide of the 3' end adapter comprises a sequencing primer sequence;
preferably, the first oligonucleotide of the 5' end adapter and/or the second oligonucleotide of the 3' end adapter further comprises a sample tag sequence; and
preferably, the first oligonucleotide of the 5' end adapter and/or the second oligonucleotide of the 3' end adapter further comprises a unique molecular identifier sequence.

12. The adapter composition according to claim 1, wherein:
the first oligonucleotide of the 5' end adapter and/or the second oligonucleotide of the 3' end adapter is immobilized on a solid phase support;
preferably, a non-ligating end of the first oligonucleotide of the 5' end adapter and/or the second oligonucleotide of the 3' end adapter is immobilized on the solid phase support; and
preferably, the solid phase support is selected from the group consisting of a bead, and a chip.

13. The adapter composition according to claim 12, wherein:
the chip is usable in a sequencing platform, for example, the chip is a sequencing chip; and
preferably, the bead is selected from the group consisting of an agarose gel bead, an agarose bead, a magnetic bead, a protein A conjugated bead, a protein G conjugated bead, a protein L conjugated bead, an oligo (dT) conjugated bead, a silica bead, a hydrogel bead, a silica-like bead, an anti-biotin microbead, an anti-fluorescent dye microbead, and any combination thereof.

14. A kit for nucleic acid library construction, comprising the adapter composition of claim 1.

15. The kit for nucleic acid library construction according to claim 14, wherein:
the kit for nucleic acid library construction further comprises: a polynucleotide kinase;
preferably, the kit for nucleic acid library construction further comprises: a PCR primer;
preferably, the kit for nucleic acid library construction further comprises: a cyclization reagent combination; and
preferably, when the nucleic acid is RNA, the kit for nucleic acid library construction further comprises: a reverse transcription primer.

16. A method for adapter ligation, comprising the following steps:
obtaining a single-stranded template nucleic acid, wherein the 5' end and the 3' end of the single-stranded template nucleic acid are both hydroxyl groups; and performing an adapter ligation reaction of a 5' end adapter and a 3' end adapter to the single-stranded template nucleic acid using a first ligase, the 5' end adapter, a second ligase, and the 3' end adapter;
wherein: the 5' end adapter comprises a first oligonucleotide, the first oligonucleotide of the 5' end adapter has a first modifying group at the 3' end, the first oligonucleotide of the 5' end adapter has a first blocking group at the 5' end, and the first modifying group comprises a phosphate group;
the first ligase is capable of ligating the first modifying group at the 3' end of the first oligonucleotide of the 5' end adapter to the 5'-hydroxyl end of the template nucleic acid;
the 3' end adapter comprises a second oligonucleotide, the second oligonucleotide of the 3' end adapter has a second blocking group at the 3' end, the second oligonucleotide of the 3' end adapter has a second modifying group at the 5' end, and the second modifying group comprises a phosphate group; and
the second ligase is capable of ligating the second modifying group at the 5' end of the second oligonucleotide of the 3' end adapter to the 3'-hydroxyl end of the template nucleic acid.

17. The method for adapter ligation according to claim 16, wherein the adapter ligation reaction is performed in a same system.

18. The method for adapter ligation according to claim 16, wherein the adapter ligation is directional, and the first oligonucleotide of the 5' end adapter has a different nucleotide sequence from the second oligonucleotide of the 3' end adapter.

19. The method for adapter ligation according to claim 16, wherein the single-stranded template nucleic acid with hydroxyl groups at both the 5' end and the 3' end is obtained by dephosphorylation treatment.

20. The method for adapter ligation according to claim 16, wherein:
the first oligonucleotide of the 5' end adapter and/or the second oligonucleotide of the 3' end adapter is immobilized on a solid phase support; and a solid phase support with a specific sequence and a single-stranded template nucleic acid is generated by contacting the solid phase support with the single-stranded template nucleic acid for ligation reaction.

21. The method for adapter ligation according to claim 16, wherein the single-stranded template nucleic acid is obtained by subjecting the nucleic acid to at least one treatment of fragment, denaturation, and DNA methylation;
preferably, the nucleic acid comprises: at least one of DNA and RNA;
preferably, the DNA comprises: at least one of dsDNA and ssDNA;
preferably, the nucleic acid is derived from the following biological samples: cells, fresh tissues, fresh organs, decayed tissues, formalin-fixed tissues, paraffin-embedded tissues, forensic samples, paleontological fossils, or biological materials containing cfDNA or cfRNA; and
preferably, the biological materials comprise: peripheral blood, plasma, serum, urine, feces, saliva, cerebrospinal fluid, lymph fluid, bronchoalveolar lavage fluid, amniotic fluid, blastocyst cavity fluid, cell culture fluid, embryo culture fluid, microbial culture medium, soil leachate, and bone meal leachate.

22. The method for adapter ligation according to claim 21, wherein, when the nucleic acid contains dsDNA, a process of preparing the single-stranded template nucleic acid comprises denaturation treatment; and
preferably, the denaturation treatment is performed before or after phosphorylation treatment.

23. The method for adapter ligation according to claim 22, wherein:
when the nucleic acid is used for constructing a DNA methylation library, the process of preparing the single-stranded template nucleic acid comprises DNA methylation treatment;
preferably, the methylation treatment is performed before the phosphorylation treatment and the denaturation treatment; and
preferably, the methylation treatment is performed after the fragment treatment.

24. A method for nucleic acid library construction, comprising the following steps:
obtaining a single-stranded template nucleic acid, wherein the 5' end and the 3' end of the single-stranded template nucleic acid are both hydroxyl groups; and performing an adapter ligation reaction of a 5' end adapter and a 3' end adapter to the single-stranded template nucleic acid using a first ligase, the 5' end adapter, a second ligase and the 3' end adapter, to obtain an adapter-ligated single-stranded template nucleic acid; and
performing an amplification reaction to obtain a nucleic acid library;
wherein: the 5' end adapter comprises a first oligonucleotide, the first oligonucleotide of the 5' end adapter has a first modifying group at the 3' end, the first oligonucleotide of the 5' end adapter has a first blocking group at the 5' end, the first modifying group comprises a phosphate group;
the first ligase is capable of ligating the first modifying group at the 3' end of the first oligonucleotide of the 5' end adapter to the 5'-hydroxyl end of the template nucleic acid;
the 3' end adapter comprises a second oligonucleotide, the second oligonucleotide of the 3' end adapter has a second blocking group at the 3' end, the second oligonucleotide of the 3' end adapter has a second modifying group at the 5' end, and the second modifying group comprises a phosphate group; and
the second ligase is capable of ligating the second modifying group at the 5' end of the second oligonucleotide of the 3' end adapter to the 3'-hydroxyl end of the template nucleic acid.

25. The method according to claim 24, wherein:
the first oligonucleotide of the 5' end adapter and/or the second oligonucleotide of the 3' end adapter is immobilized on a solid phase support; and a solid phase support with a specific sequence and a single-stranded template nucleic acid is generated by contacting the solid phase support with the single-stranded template nucleic acid for ligation reaction;
preferably, when the nucleic acid is RNA, the method further comprises a step of performing a reverse transcription reaction;
preferably, the method further comprises a step of performing a cyclization reaction after the adapter ligation reaction; and
preferably, the nucleic acid library comprises a DNA methylation library.

26. The method according to claim 24, wherein the amplification reaction is a rolling circle amplification or a linear amplification.

27. A nucleic acid library, which is obtained by the following steps:
obtaining a single-stranded template nucleic acid, wherein the 5' end and the 3' end of the single-stranded template nucleic acid are both hydroxyl groups; and performing an adapter ligation reaction of a 5' end adapter and a 3' end adapter to the single-stranded template nucleic acid using a first ligase, the 5' end adapter, a second ligase and the 3' end adapter, to obtain an adapter-ligated single-stranded template nucleic acid; and
performing an amplification reaction to obtain a nucleic acid library;
wherein: the 5' end adapter comprises a first oligonucleotide, the first oligonucleotide of the 5' end adapter has a first modifying group at the 3' end, the first oligonucleotide of the 5' end adapter has a first blocking group at the 5' end, and the first modifying group comprises a phosphate group;
the first ligase is capable of ligating the first modifying group at the 3' end of the first oligonucleotide of the 5' end adapter to the 5'-hydroxyl end of the template nucleic acid;
the 3' end adapter comprises a second oligonucleotide, the second oligonucleotide of the 3' end adapter has a second blocking group at the 3' end, the second oligonucleotide of the 3' end adapter has a second modifying group at the 5' end, and the second modifying group comprises a phosphate group; and
the second ligase is capable of ligating the second modifying group at the 5' end of the second oligonucleotide of the 3' end adapter to the 3'-hydroxyl end of the template nucleic acid.
